# EUROPEAN PATENT APPLICATION

(11) **EP 3 919 077 A1**
(43) Date of publication of application: **08.12.2021**
(21) Application number: 20178287.7
(22) Date of filing: 04.06.2020
(51) Int. Cl.: A61K 39/395, A61P 35/00, A61P 37/06, C07K 14/575, C07K 14/72, C07K 16/00, C07K 16/28, A61K 39/00

(54) **MODEL FOR PREDICTION OF TOLERABILITY ISSUES IN CONNECTION WITH INTRAVENOUS ADMINISTRATION OF THERAPEUTIC ANTIBODIES**

(71) Applicant: BioInvent International AB, 223 70 Lund (SE)
(72) Inventor: Frendéus, Björn, 224 68 Lund (SE); Mårtensson, Linda, 237 91 Bjärred (SE); Karlsson, Ingrid, 226 51 Lund (SE); Teige, Ingrid, 224 68 Lund (SE)
(74) Representative: Potter Clarkson

(57) **Abstract**

Described herein is a model for predicting if a therapeutic antibody binding to a human target will be associated with a tolerability issue in connection with intravenous administration and/or for predicting if pre-treatment, altered administration route or modification of the antibody can prevent a tolerability issue associated with intravenous administration to a human of the therapeutic antibody. The model comprises administering the antibody intravenously or intraperitoneally to mice and observing the mice immediately after the administration for any transient display of the macroscopic symptoms isolation and decreased activity. The model may also comprise administration of a pre-treatment in combination with administration of the antibody, administration of the therapeutic antibody by a route of administration other than intravenous or intraperitoneal administration or administration of a modified format of the antibody to mice and observing the mice immediately after such administration for any transient display of the macroscopic symptoms isolation and decreased activity and comparing this with the transient display of the macroscopic symptoms isolation and decreased activity after the intravenous or intraperitoneal administration of the unmodified antibody without pre-treatment.

## Description

### FIELD OF THE INVENTION

The present invention relates to a method or model that can be used to predict if a therapeutic antibody molecule binding specifically to a human target will be associated with a tolerability issue in connection with intravenous administration to a human, and/or to predict if a prophylactic or therapeutic treatment, an altered administration route and/or a modification of the therapeutic antibody molecule can prevent or mitigate a tolerability issue associated with intravenous administration to a human of a therapeutic antibody molecule binding specifically to a human target.

### BACKGROUND OF THE INVENTION

Therapeutic antibodies constitute a well-proven class of drugs, currently comprising xxx antibodies approved for therapy of diverse diseases e.g. cancer, inflammatory-, autoimmune- and infectious disease. Several monoclonal antibody therapies, in particular those used for cancer therapy, are administered by intravenous infusion allowing high immediate drug exposure that can be maintained through repeated dosing. In many cases, the patient may then experience an infusion-related reaction (IRR). An IRR is caused by an adverse reaction to the infusion of the therapeutic antibody. IRRs include hypersensitivity reactions and cytokine release syndromes. IRRs are experienced by patients during the infusion of the monoclonal antibody (uniphasic reaction) and/or within hours of the infusion (biphasic or delayed reaction), and they include hypersensitivity reactions and cytokine release syndromes. Common IRRs include but are not limited to respiratory conditions such as nasal congestion, cough, allergic rhinitis, throat irritation, and dyspnea and non-respiratory conditions such as chills and nausea. Often the IRRs occur with the first dose administered to a patient, but they can also occur after the second or subsequent administration. In many cases the IRRs are mild, but more serious IRRs can sometimes occur which risk being fatal if not managed appropriately. An IRR may affect any organ system in the body.

According to Common Terminology Criteria for Adverse Events (CTCAE) version 5.0 published November 27, 2017 by the U.S. Department of Health and Human Services, the severity of adverse events, such as IRRs, is categorized in different grades, ranging from 1 (the least severe) to 5 (the most severe).

Management of the toxicities associated with cancer immunotherapy is a challenging clinical problem. Severe CRS can represent a life-threatening adverse event that requires prompt and aggressive treatment. Reduction of tumor burden, limitations on the dose of administered therapy, and premedication with steroids have reduced the incidence of severe CRS, as have the use of anti-cytokine treatments.

The conventional management of hypersensitivity reactions, such as IRRs, includes temporary interruption of infusion, lowering the infusion rate, and/or treatment with antihistamines, antipyretics, and/or corticosteroids or in severe case interruption / cessation of infusions. In such severe cases, cautious re-introduction of infusions, at a slower rate with increases as tolerated may be considered. Pre-treatment with antipyretics and/or antihistamines may prevent reactions subsequent infusions.

Corticosteroids are often used to prevent or dampen infusion related reactions (IRR's) and associated toxicities seen with therapeutic antibodies. The corticosteroid regimen, i.e. type of corticosteroid, dose, and timing of administration, depends both on the therapeutic antibody of use and on the indication. Rituxan (rituximab) is a CD20-directed cytolytic antibody commonly used both in CD20-positive B cell lymphomas (Non-Hodgkin's Lymphoma (NHL) and Chronic Lymphocytic Leukemia (CLL)) as well as chronic inflammatory disorders such as Rheumatoid Arthritis (RA). In the case of NHL and CLL, corticosteroids are often used to lower the risk of IRR's and then administrated 30 minutes prior to the first rituximab cycle, and only at subsequent cycles if severe infusion-related adverse events were experienced during the first cycle. In the case of NHL corticosteroids (i.e. prednisone) are also used as part of in combination therapy. i.e. rituximab, cyclophosphamide, doxorubicin, vincristine, and prednisone (R-CHOP). In the case of RA, corticosteroid is recommended 30 minutes prior to each infusion. When administering another CD20-directed antibody Gazyva (obinutuzumab), corticosteroid premedication is also recommended prior to the first treatment cycle and prior to following cycles only in patients where grade 3 IRR was experienced with the previous infusion or with a lymphocyte count > 25 x 109/L prior to next treatment. In the case of Gazyva corticosteroid premedication should be given at least 1 hour prior to the antibody infusion. A third example of a therapeutic antibody where corticosteroids are used to lower the risk of IRR's is Darzalex (daratumumab), a CD38-directed antibody indicated for the treatment of patients with multiple myeloma. Corticosteroids are in this case recommended pre and post every infusion, 1-3 hour prior to infusion and then again on each of the 2 days following infusion.

WO 2020/047389 describes dosing strategies and administration regimens for therapeutic protein, such as antibodies (e.g., bispecific antibodies targeting T cells), that mitigate the prevalence and severity of cytokine release syndrome or an infusion-related reaction in patients undergoing immunotherapy. comprising: (i) administering fractions of a primary dose (D1) of the therapeutic protein in week 1 of the dosing regimen, wherein the primary dose comprises no more than 10 mg of the therapeutic protein, a first dose fraction (F1D1) comprises 40% to 60% of the total primary dose and is administered to the subject on day 1 of week 1, and a second dose fraction (F2D1) comprises the remaining 40% to 60% of the total primary dose and is administered to the subject from 12 to 96 hours following administration of the F1D1; (ii) administering fractions of a secondary dose (D2) of the therapeutic protein in week 2 of the dosing regimen, wherein the secondary dose is no more than one-half of a maximum weekly dose of the therapeutic protein, a first dose fraction (F1D2) comprises 40% to 60% of the total secondary dose, a second dose fraction (F2D2) comprises the remaining 40% to 60% of the total secondary dose, and the F2D2 is administered to the subject from 12 to 96 hours following administration of the F1D2 during week 2 of the dosing regimen; and (iii) administering the maximum weekly dose of the therapeutic protein to the subject as a single dose in a subsequent week of the dosing regimen. Fractionated dosing is not ideal, since dosing with suboptimally efficacious doses risk limiting therapeutic benefit, in the worst case resulting in no clinical benefit of the intended therapeutic treatment or induction of disease progression.

WO 2020/037024 mentions the use of an additional therapeutic agent, such as an antihistamine, acetaminophen or a corticosteroid, to prevent or reduce the severity of adverse events, such as an infusion-related reaction, in treatment of ovarian cancer, peritoneal cancer or fallopian tube cancer with an anti-tissue factor antibody or an antigen-binding fragment thereof conjugated to a monomethyl auristatin or a functional analog or derivative thereof or a functional derivative thereof.

The above demonstrates that intravenous administration of antibodies to different targets often is associated with tolerability issues. Such tolerability issues may vary between different therapeutic antibodies and between patients with varying frequency duration, severity and being of different nature. Accordingly, methods to reduce, suppress or overcome the different tolerability issues associated with iv administration of different antibodies differ greatly, and comprise administration of different agents e.g. corticosteroids or antihistamines immediately before, concomitantly, and/or following intravenous administration of the therapeutic antibody. Given the heterogeneity in nature and frequency of tolerability issues associated with antibodies to different targets, and the poor molecular and cellular understanding of mechanisms underlying these, there is a great need and value of methods enabling prediction of whether or not tolerability issues are likely to occur in association with intravenous administration of antibodies to different targets, and equally importantly of methods enabling discovery of means that help prevent, suppress or overcome tolerability issues associated with iv administration of antibodies to given targets. Preclinical methods allowing for such prediction and screening at early stages of therapeutic antibody development at a relatively lower cost and higher throughput compared to the human clinical setting are of outstanding importance.

### SUMMARY OF THE INVENTION

Disclosed herein is a method (or model) for predicting if a therapeutic antibody molecule binding specifically to a human target will be associated with a tolerability issue in connection with intravenous administration to a human, comprising the following step:
(i) intravenous or intraperitoneal administration of the therapeutic antibody molecule, if cross-reactive with murine target, or a surrogate antibody, to a mouse and observation of the mouse during a period following immediately after the administration of the therapeutic or surrogate antibody, wherein a display of the macroscopic symptoms isolation and decreased activity during the period followed by restoration of the state of the mouse to the normal state is an indication that the intravenous administration of the therapeutic antibody molecule to a human will be associated with a tolerability issue,
and/or for predicting if a prophylactic or therapeutic treatment, an altered administration route and/or a modification of the therapeutic antibody molecule can prevent or mitigate a tolerability issue associated with intravenous administration to a human of a therapeutic antibody molecule binding specifically to a human target,
comprising the following step(s) in addition to (i) as set out above:
(ii) administration of a prophylactic or therapeutic agent to a mouse in conjunction with intravenous or intraperitoneal administration of the therapeutic or surrogate antibody to a mouse, and observation of the mouse during a period following immediately after the administration of the therapeutic or surrogate antibody, wherein a decreased display of the macroscopic symptoms compared to the macroscopic symptoms displayed by the mouse in (i) or no display of the macroscopic symptoms during the period is an indication that pre-treatment with the prophylactic or therapeutic agent in combination with administration of the therapeutic antibody molecule to a human can prevent or mitigate the tolerability issue that otherwise would be associated with intravenous administration of the therapeutic antibody molecule to a human;
(iii) administration of the therapeutic or surrogate antibody to a mouse by a route of administration other than intravenous or intraperitoneal administration, and observation of the mouse during a period following immediately after the administration of the therapeutic or surrogate antibody, wherein a decreased display of the macroscopic symptoms compared to the macroscopic symptoms displayed by the mouse in (i) or no display of the macroscopic symptoms during the period is an indication that the other route of administration can be used for administration of the therapeutic antibody molecule to a human to prevent or mitigate the tolerability issue that would be associated with intravenous administration of the therapeutic antibody molecule to a human; and/or
(iv) intravenous or intraperitoneal administration of a modified format of the therapeutic or surrogate antibody to a mouse by a route of administration other than intravenous or intraperitoneal administration, and observation of the mouse during a period following immediately after the administration of the modified therapeutic or surrogate antibody, wherein a decreased display of the macroscopic symptoms compared to the macroscopic symptoms displayed by the mouse in (i) or no display of the macroscopic symptoms during the period is an indication that administration of the therapeutic antibody molecule in the modified format to a human can be used to prevent or mitigate the tolerability issue that would be associated with intravenous administration of the therapeutic antibody molecule to a human.

Disclosed herein is also a corticosteroid for use in a dosing regimen to prevent or mitigate a tolerability issue in connection with intravenous administration of a therapeutic antibody molecule to a subject,
wherein the therapeutic antibody molecule has been predicted to be associated with a tolerability issue in connection with intravenous administration to a human using the method described above, and/or wherein pre-treatment with the corticosteroid combination with administration of the therapeutic antibody molecule to a human has been predicted to prevent or mitigate the tolerability issue that otherwise would be associated with intravenous administration of the therapeutic antibody molecule to a human using the method described above,
and wherein the dosing regimen comprises administration of the corticosteroid to the subject in at least two doses prior to intravenous administration of the therapeutic antibody molecule, wherein one dose of the corticosteroid is administered 10-48 hours prior to start of the administration of therapeutic antibody molecule ("the first dose") and one dose of the corticosteroid is administered 5 minutes - 5 hours prior to the start of administration of the therapeutic antibody molecule ("the second dose").

Disclosed herein is also a therapeutic antibody molecule for use in the treatment of cancer, wherein the therapeutic antibody molecule has been predicted to be associated with a tolerability issue in connection with intravenous administration to a human using the method described above and/or wherein the subcutaneous route of administration of therapeutic antibody molecule to a human has been predicted to prevent or mitigate the tolerability issue that otherwise would be associated with intravenous administration of the therapeutic antibody molecule to a human using the method described above, and wherein the antibody is formulated for subcutaneous administration.

Disclosed herein is also a modified format of a therapeutic antibody molecule for use in the treatment of cancer, wherein the therapeutic antibody molecule has been predicted to be associated with a tolerability issue in connection with intravenous administration to a human using the method described above and/or wherein administration of the therapeutic antibody molecule in the modified format to a human has been predicted to prevent or mitigate the tolerability issue that otherwise would be associated with intravenous administration of the therapeutic antibody molecule to a human using the method described above, and wherein the therapeutic antibody molecule is an Fc receptor binding antibody and the modified format is an antibody having the same Fv variable sequence but having impaired or abrogated FcγR binding compared with the therapeutic antibody molecule.

Disclosed herein is also a method for preventing or mitigating a tolerability issue in connection with intravenous administration of a therapeutic antibody molecule to a subject comprising a corticosteroid dosing regimen, wherein the therapeutic antibody molecule has been predicted to be associated with a tolerability issue in connection with intravenous administration to a human using the predictive method described above, and/or wherein pre-treatment with the corticosteroid combination with administration of the therapeutic antibody molecule to a human has been predicted to prevent or mitigate the tolerability issue that otherwise would be associated with intravenous administration of the therapeutic antibody molecule to a human using the predictive method described above, and wherein the dosing regimen comprises administration of the corticosteroid to the subject in at least two doses prior to intravenous administration of the therapeutic antibody molecule, wherein one dose of the corticosteroid is administered 10-48 hours prior to start of the administration of therapeutic antibody molecule ("the first dose") and one dose of the corticosteroid is administered 5 minutes - 5 hours prior to the start of administration of the therapeutic antibody molecule ("the second dose").

Disclosed is also a method for treatment of cancer comprising subcutaneous administration of a therapeutically active amount of a therapeutic antibody molecule which has been predicted to be associated with a tolerability issue in connection with intravenous administration to a human using the predictive method described above and/or wherein the subcutaneous route of administration of therapeutic antibody molecule to a human has been predicted to prevent or mitigate the tolerability issue that otherwise would be associated with intravenous administration of the therapeutic antibody molecule to a human using the predictive method described above.

Disclosed is also a method for treatment of cancer comprising administration of a therapeutically active amount of a modified format of a therapeutic antibody, wherein the therapeutic antibody molecule has been predicted to be associated with a tolerability issue in connection with intravenous administration to a human using the predictive method described above and/or wherein administration of the therapeutic antibody molecule in the modified format to a human has been predicted to prevent or mitigate the tolerability issue that otherwise would be associated with intravenous administration of the therapeutic antibody molecule to a human using the predictive method described above, and wherein the therapeutic antibody molecule is an Fc receptor binding antibody and the modified format is an antibody having the same Fv variable sequence but having impaired or abrogated FcyR binding compared with the therapeutic antibody molecule.

### DETAILED DESCRIPTION OF THE INVENTION

In brief, we describe a model for predicting if a therapeutic antibody binding to a human target will be associated with a tolerability issue in connection with intravenous administration and/or for predicting if pre-treatment, altered administration route or modification of the antibody can prevent a tolerability issue associated with intravenous administration to a human of the therapeutic antibody. The model comprises administering the antibody intravenously or intraperitoneally to mice and observing the mice immediately after the administration for any transient display of the macroscopic symptoms isolation and decreased activity. The model may also comprise administration of a pre-treatment in combination with administration of the antibody, administration of the therapeutic antibody by a route of administration other than intravenous or intraperitoneal administration or administration of a modified format of the antibody to mice and observing the mice immediately after such administration for any transient display of the macroscopic symptoms isolation and decreased activity and comparing this with the transient display of the macroscopic symptoms isolation and decreased activity after the intravenous or intraperitoneal administration of the unmodified antibody without pre-treatment.

The predictive method described herein makes it possible to predict if a therapeutic antibody molecule to a given target will be associated with - or is likely to be associated with - a tolerability issue in connection with its intravenous administration to a human subject. Additionally or alternatively, it makes it possible to predict if a prophylactic or therapeutic treatment, an altered administration route and/or a modification of the therapeutic antibody molecule can be used to prevent or mitigate a tolerability issue associated with intravenous administration to a human of a therapeutic antibody molecule binding specifically to a human target.

The therapeutic antibody molecule binds specifically to a human target. That the antibody binds specifically to the target means that it specifically binds to or interacts with a defined target molecule or antigen, and that this means that the antibody preferentially and selectively binds its target and not a molecule which is not a target.

The target to which the therapeutic antibody molecule binds may be a receptor or antigen found on any human cell. Examples of such cells are leukocytes, myeloid cells and B cells.

In some embodiments the target is FcγRII (CD32).

In some embodiments the target is FcγRIIB (CD32b).

In some embodiments the target is FcγRIIA (CD32a).

In some embodiments the target is CD40.

The therapeutic antibody molecule may be any therapeutic antibody molecule that has received regulatory approval for use in humans or a therapeutic antibody molecule in clinical development or may be any antibody binding to a human target antigen intended or hypothesized to be of use for therapy of human disease. The term therapeutic antibody molecule as used herein also encompasses antibodies that are envisaged to be considered for, or are being developed for, therapeutic use, including antibodies in preclinical development. The therapeutic antibody molecule is thus an antibody that has therapeutic effects on humans. In the predictive method described herein, the therapeutic antibody molecule (if cross-reactive), or a surrogate antibody to the analogous mouse target, may be administered to a mouse. The mouse used is an immune competent laboratory mouse. Mice of different genetic background, inbred or outbred, may be used. Further, mice transgenic for the human target of the therapeutic antibody molecule may be used.

Often, such a therapeutic antibody molecule is a monoclonal antibody. In many cases it is a human or humanized antibody.

The therapeutic antibody molecule may be any type of antibody, such as immunoglobulin G (IgG), immunoglobulin A (IgA) or immunoglobulin M (IgM). In some embodiments, it is IgG. It may also be of any subclass, such as IgG1, IgG2, IgG3 or IgG4. It may also be an antibody molecule engineered for enhanced or diminished FcγR-dependent engagement and function. Further, the therapeutic antibody molecule may be a mono-, bi- or tri-specific for the same or different targets and comprising or being fused to an antibody Fc domain. Moreover, the therapeutic antibody molecule may be a mono-, bi- or tri-specific for the same or different targets and not comprising an antibody Fc domain. Further, the therapeutic antibody molecule may be a functional fragment of an antibody, such as an Fv, consisting of the variable domain of an antibody, a Fab, also denoted F(ab), which is a monovalent antigen-binding fragment that does not contain a Fc part, or a F(ab')₂, which is an divalent antigen-binding fragment that contains two antigen-binding Fab parts linked together by disulfide bonds, or a F(ab'), i.e. a monovalent-variant of a F(ab')₂. Such a fragment may also be single chain variable fragment (scFv).

In some embodiments, the therapeutic antibody molecule is an antibody used in or intended for cancer therapy. Monoclonal antibodies have been and are being developed for a number of cancers, and more will likely follow. Some examples are brain cancer, breast cancer, chronic lymphocytic leukemia, colorectal cancer, head and neck cancers, Hodgkin's lymphoma, lung cancer, melanoma, non-Hodgkin's lymphoma, prostate cancer and stomach cancer. The method described herein is however not limited to antibodies used in cancer therapy.

As mentioned above, in some embodiments the target is FcγRIIB. The therapeutic antibody molecule may then be an antibody molecule described in WO 2012/022985, WO 2015/173384 and/or WO 2019/138005. In some embodiments it is an antibody having the CDR sequences SEQ ID Nos: 83-88 as described in WO 2012/022985. In some embodiments, it is an antibody having a VH with Seq ID No: 12 and a VL with Seq ID No: 25 as described in WO 2012/022985. In some embodiments, it is the antibody described in WO 2012/022985 as having a VH with Seq ID No: 12, a VL with Seq ID No: 25; a CH with Seq ID No: 1 and a CL with Seq ID No: 2 (corresponding to the antibody disclosed herein with a light chain having SEQ. ID. No: 1 and a heavy chain having SEQ. ID. No: 2).

The intravenous (iv) administration method used to administer the therapeutic antibody molecule to a human may be any type of intravenous administration, such as by injection or by infusion.

The term tolerability issue as used herein encompasses different types of adverse events that may occur in connection with intravenous administration of the therapeutic antibody molecule to a human. These may be, for example, IRRs, cytokine release syndrome, thrombocytopenia, hepatic toxicities such as elevated liver enzymes, fever, hypotension and/or dermatological toxicities, including rashes such as urticaria. Herein these different tolerability issues are defined in the way they are defined in the CTCAE version 5.0, as further described below.

Tolerability issues may be of different grades, i.e. of different severity for the patients experiencing them. In some cases, they lead to discomfort for the patient, while in others they may cause severe problems that may prevent continued treatment with the therapeutic antibody molecule. In worse cases, the tolerability issues may even lead to death of the patient.

The tolerability issues that may be predicted and/or prevented or mitigated as described herein are adverse events that occur in connection with intravenous administration of the therapeutic antibody molecule to a patient, i.e. immediately when the therapeutic antibody molecule is administered, such as within a few minutes up to a few hours or within 24 hours from administration of the therapeutic antibody molecule to the patient. In many cases, the first tolerability issues are observed within less 30 minutes.

In some cases, it may be of interest to prevent in particular thrombocytopenia and/or hepatic toxicities.

The IRR that may be predicted with the method described herein may be any IRR. The adverse event denoted "Infusion related reaction" in the CTCAE version 5.0 is used for disorders characterized by adverse reaction to the infusion of pharmacological or biological substances; belonging to the group of "Injury, poisoning and procedural complications". The five grades identified in the CTCAE are the following:
1) Mild transient reaction; infusion interruption not indicated; intervention not indicated
2) Therapy or infusion interruption indicated but responds promptly to symptomatic treatment (e.g., antihistamines, NSAIDS, narcotics, IV fluids); prophylactic medications indicated for ≤24 hours
3) Prolonged (e.g., not rapidly responsive to symptomatic medication and/or brief interruption of infusion); recurrence of symptoms following initial improvement; hospitalization indicated for clinical sequelae
4) Life-threatening consequences; urgent intervention indicated
5) Death.

The adverse event denoted "Cytokine release syndrome" in the CTCAE version 5.0 is used for disorders characterized by fever, tachypnea, headache, tachycardia, hypotension, rash, and/or hypoxia caused by the release of cytokines, belonging to the group of "Immune system disorders". The five grades identified in the CTCAE are the following:
1) Fever with or without constitutional symptoms
2) Hypotension responding to fluids; hypoxia responding to <40% O₂
3) Hypotension managed with one pressor; hypoxia requiring ≥ 40% O₂
4) Life-threatening consequences; urgent intervention indicated
5) Death

The adverse event denoted "Platelet count decreased" (i.e. thrombocytopenia) in the CTCAE version 5.0 is used for findings based on laboratory test results that indicate a decrease in number of platelets in a blood specimen, belonging to the group of "Investigations". The five grades identified in the CTCAE are the following:
1) <LLN - 75,000/mm3; <LLN - 75.0 x 10e9 /L
2) <75,000 - 50,000/mm3; <75.0 - 50.0 x 10e9 /L
3) <50,000 - 25,000/mm3; <50.0 - 25.0 x 10e9 /L
4) <25,000/mm3; <25.0 x 10e9 /L
5) -

The associated toxicity may also be a hepatic adverse event or hepatic toxicities. Examples of such toxicities are an elevation of one or both of the two enzymes aspartate aminotransferase (AST) and alanine aminotransferase (ALT). Like thrombocytopenia, the adverse events denoted "Aspartate aminotransferase increased" and "Alanine aminotransferase increased" in the CTCAE version 5.0 belong to the group of "Investigations". Increased AST or ALT, respectively, is a finding based on laboratory test results that indicate an increase in the level of AST (or SGOT) and ALT (or SGPT), respectively, in a blood specimen. The five grades identified in the CTCAE both for increased AST and increased ALT are the following:
1) >ULN - 3.0 x ULN if baseline was normal; 1.5 - 3.0 x baseline if baseline was abnormal
2) >3.0 - 5.0 x ULN if baseline was normal; >3.0 - 5.0 x baseline if baseline was abnormal
3) >5.0 - 20.0 x ULN if baseline was normal; >5.0 - 20.0 x baseline if baseline was abnormal
4) >20.0 x ULN if baseline was normal; >20.0 x baseline if baseline was abnormal
5) -.

The adverse event denoted "Fever" in the CTCAE version 5.0 is used for disorders characterized by elevation of the body's temperature above the upper limit of normal, belonging to the group of "General disorders and administration site conditions". The five grades identified in the CTCAE are the following:
1) 38.0 - 39.0 °C
2) >39.0 - 40.0 °C
3) >40.0 °C for ≤24 hours
4) >40.0 °C for >24 hours
5) Death.

The adverse event denoted "Hypotension" in the CTCAE version 5.0 is used for disorders characterized by a blood pressure that is below the normal expected for an individual in a given environment, belonging to the group of "Vascular disorders". The five grades identified in the CTCAE are the following:
1) Asymptomatic, intervention not indicated
2) Non-urgent medical intervention indicated
3) Medical intervention indicated; hospitalization indicated
4) Life-threatening consequences and urgent intervention indicated
5) Death.

The adverse event denoted "Urticaria" in the CTCAE version 5.0 is used for disorders characterized by an itchy skin eruption characterized by wheals with pale interiors and well-defined red margins, belonging to the group of "Skin and subcutaneous tissue disorders". The five grades identified in the CTCAE are the following:
1) Urticarial lesions covering <10% BSA; topical intervention indicated
2) Urticarial lesions covering 10 - 30% BSA; oral intervention indicated
3) Urticarial lesions covering >30% BSA; IV intervention indicated
4) -
5) -.

The predictive method described herein utilizes mice as a model to predict what will happen in humans. When the therapeutic antibody molecule to be tested is cross reactive with a known murine homologue of the human target, the therapeutic antibody molecule may be used in the predictive method. When the therapeutic antibody molecule to be tested is not cross reactive with a known murine homologue of the human target, it is necessary to use a surrogate antibody. The surrogate antibody is an antibody that is specific for a murine homologue of the human target to which the therapeutic antibody molecule binds. For example, in this context the murine homologue of the human target FcγRIIB is murine FcγRII, the murine homologue of the human target FcγRIIA is murine FcγRIII, and the murine homologue of the human target CD40 is murine CD40. The surrogate antibody may be a murine antibody or an antibody from another species, such as rat, rabbit, monkey or chicken. Sometimes, it may be preferable to use a surrogate antibody even if the therapeutic antibody molecule to be tested is cross reactive with a known murine homologue of the human target. This may be the case if the surrogate antibody's binding and interaction with mouse target antigen and mouse immune proteins regulating antibody activity e.g. FcγRs better reflect the interactions of the human candidate antibodies interaction with human target and human immune proteins regulating antibody activity e.g. FcγRs compared with the therapeutic antibody molecule itself. Preferably, and when available, both cross-reactive therapeutic antibody molecule and murine surrogate antibodies, may be used in parallel to test for tolerability issues as described herein.

The therapeutic antibody molecule may be an antibody that binds or does not bind Fc receptors. If a surrogate antibody is used to predict or model tolerability issues of a therapeutic antibody molecule to the same or homologous target, then the Fc of the surrogate antibody should be chosen to match the therapeutic antibody molecule's Fc with respect to binding/non-binding (or engagement/non- engagement) of FcγR-binding and function. It is, for example, well known that both human IgG1, IgG3 and IgG4 productively bind and engage human FcγRs albeit with different absolute and relative affinities. Similarly, in the mouse, mlgG2a binds strongly and broadly to different mouse FcγRs, while mlgG1 binds only to mouse FcγRII and FcγRIII. It is further well known aglycosylation of antibodies, specifically in the 297 position (such as one of the following mutations: N297A, N297Q or N297G), render both human and mouse IgG impaired for binding to FcγR. In this context, Fc binding means that the Fc part of the therapeutic antibody molecule binds to an FcγR which leads to engagement of Fc:FcγR dependent activities or functions. Furthermore, impaired or abrogated FcγR binding means that the modified format does not bind at all to FcγR or that it binds less strongly to FcγR than the therapeutic, unmodified antibody.

The therapeutic antibody molecule or the surrogate antibody is administered intravenously or intraperitoneally to a mouse. In some cases, the dose of the therapeutic antibody molecule or the surrogate antibody administered to the mouse is the dose that results in high receptor saturation. In some cases, the dose of the therapeutic antibody molecule or the surrogate antibody administered to the mouse is the dose that results in at least 90% receptor saturation. In some cases, the dose of the therapeutic antibody molecule or the surrogate antibody administered to the mouse is the dose that results in close to 100% or 100% receptor saturation.

Once the antibody has been administered to the mouse the animal is observed for visual physical reactions, in particular behavior changes or macroscopic symptoms, are observed. If the therapeutic antibody molecule is an antibody that will be associated with a tolerability issue in connection with intravenous administration to a human, the mouse will starting to show the macroscopic symptoms isolation and decreased activity very shortly, i.e. within a few minutes, such as 5-10 minutes, after administration of the therapeutic or surrogate antibody. In some cases, the mouse will also display signs of impaired balance, piloerection, and/or hunching followed by un-natural body posture. These three additional macroscopic symptoms will be observed within the same time frame as the isolation and decreased activity, i.e. within a few minutes, such as 5-10 minutes, after administration of the therapeutic or surrogate antibody. The display of one, two or three of these additional macroscopic symptoms is a stronger predictive marker that the therapeutic antibody molecule binding specifically to a human target is likely to be associated with a tolerability issue in connection with intravenous administration to a human compared to if the mouse would only show signs of isolation and decreased activity.

A person experienced in working with laboratory mice will immediately notice if the above changes occurs in the mouse's behavior, since the signs are easy to observe and is a notable change of the mouse behavior prior to administration of the therapeutic or surrogate antibody. The symptoms are clearly manifested, and it is obvious that the mouse is not feeling well. After a period ending in about one hour, such as 45 minutes to 1.5 hours, after injection of the antibody (therapeutic or surrogate), the mouse no longer shows any macroscopic symptoms. Instead, its behavior is restored to the normal state, i.e. to the behavior prior to administration of the antibody (therapeutic or surrogate).

In addition to the above macroscopic symptoms, the mouse may demonstrate other symptoms. One such symptom is decreased blood pressure. Another such symptom is decreased platelet count. Another such symptom is elevated levels of the two liver enzymes aspartate aminotransferase (AST) and alanine aminotransferase (ALT). Contrary to the macroscopic symptoms, these cannot be determined by simply observing the mouse. Instead they may be determined through blood analysis. To check these "non-macroscopic" symptoms, blood is drawn from the mouse approximately five minutes after injection of the antibody (therapeutic or surrogate). The blood is then analyzed for platelet count and/or levels of AST and/or ALT. Decreased blood pressure may also be determined this way; if the blood pressure is decreased, it will not be possible to draw blood from the mouse during the period during which the macroscopic symptoms are displayed. To determine if the platelet count is decreased and/or if the level of AST and/or ALT is elevated, it is possible to compare either with a blood sample drawn from the mouse prior to administration of the therapeutic antibody molecule or surrogate antibody or with a sample drawn from a control mouse. The decreased blood pressure will be restored within the same period as the macroscopic symptoms. The platelet count, AST level and ALT level will take a bit longer to be restored; it is normalized within 6-10 hours, such as within 8 hours.

The predictive method described herein can be used to predict if a therapeutic antibody molecule binding specifically to a human target will be associated with a tolerability issue in connection with intravenous administration to a human.

Moreover, the predictive method described herein can be used test strategies for overcoming such a tolerability issue. More precisely, it can be used to predict if a specific strategy can prevent or mitigate a tolerability issue associated with intravenous administration to a human of a therapeutic antibody molecule binding specifically to a human target. This is useful for example for a therapeutic antibody molecule that is already used in the clinic for which tolerability issues have been observed.

Furthermore, the predictive method described herein can be used both to predict if a therapeutic antibody molecule binding specifically to a human target will be associated with a tolerability issue in connection with intravenous administration to a human and to predict if a specific strategy can prevent or mitigate the tolerability issue. This may be of interest for example when developing a drug since it enables both identification of a potential problems and means of finding a solution to the problem.

If the predictive method described herein is to be used only to predict if a therapeutic antibody molecule binding specifically to a human target will be associated with a tolerability issue in connection with intravenous administration to a human, the method is performed as described above, with administration of the therapeutic or surrogate antibody followed by observance of the mouse. Normally, not only one mouse would be used, but instead a test group of several mice, such as 5-10, would be used and the experiment would be repeated to ascertain that any change observed is representative, reproducible and statistically significant.

If the predictive method described herein is to be used only to, or in addition to, predict if a specific strategy can prevent or mitigate a tolerability issue associated with intravenous administration to a human of a therapeutic antibody molecule binding specifically to a human target, the method described above is performed on a control mouse, or preferably on a control group of mice, such as 5-10 mice. In addition, a second mouse, or preferably a second group of mice, such as 5-10 mice, is treated in accordance with the specific strategy that is to be tested. The results for the second mouse, or second group of mice, are compared to the results for the control mouse, or control group of mice.

Examples of strategies for overcoming tolerability issue arising in connection with intravenous administration of a therapeutic antibody molecule to a human are different prophylactic treatments, different therapeutic treatments, altered administration routes and/or modifications of the therapeutic antibody molecule. By testing such strategies as described herein, data will be obtained that can be used to predict if that particular strategy can be used to prevent or mitigate a tolerability issue that would be associated with intra-venous administration of a specific therapeutic antibody molecule to a human. Thus, it is possible to obtain reliable data without having to test the effect of different strategies on humans that first have to experience the tolerability issue(s).

When the strategy for overcoming a tolerability issue is a prophylactic treatment, a prophylactic agent is administered to the second mouse, or second group of mice, prior to the intravenous or intraperitoneal administration of the therapeutic or surrogate antibody to the mouse, or group of mice. This strategy is thus pre-treatment with the prophylactic agent. The second mouse, or second group of mice, is observed during a period following immediately after the administration of the therapeutic or surrogate antibody The results for the second mouse, or second group of mice, are compared to the results for the control mouse, or control group of mice, which has not received the prophylactic agent. A decreased display of the macroscopic symptoms for the second mouse, or the second group of mice, compared to the control mouse, or control group of mice, or no display of the macroscopic symptoms at all for the second mouse, or second group of mice, during the period is an indication that administration of the prophylactic agent can be used to prevent or mitigate the tolerability issue that would be associated with intravenous administration of the therapeutic antibody molecule to a human.

The prophylactic agent tested this way may be any agent that is known to prevent or mitigate tolerability issues, or is hypothesized, or screened, for its ability to help mitigate tolerability issues.

In some embodiments, the prophylactic treatment is pre-treatment with a corticosteroid. In some such embodiments, the pre-treatment comprises two administrations of a corticosteroid. The corticosteroid is preferably a potent corticosteroid, and more preferably a corticosteroid with as high potency as possible or as available. Examples of such corticosteroids are dexamethasone and betamethasone.

When a pre-treatment with a corticosteroid, such as dexamethasone or betamethasone, is used it may comprise two administrations of the corticosteroid prior to administration of the therapeutic or surrogate antibody. In some such embodiments, one dose of corticosteroid is administered 10-48 hours prior to administration of the therapeutic or surrogate antibody, and the other is administered 5 minutes - 5 hours prior to administration of the therapeutic or surrogate antibody. In some such embodiments, one dose of corticosteroid is administered 6-36 hours prior to administration of the therapeutic or surrogate antibody, and the other is administered 15-120 minutes prior to administration of the therapeutic or surrogate antibody. In some such embodiments, the first dose of corticosteroid is administered 16-24 hours prior to administration of the therapeutic or surrogate antibody. In some such embodiments, the second dose of corticosteroid is administered 30-60 minutes prior to administration of the therapeutic or surrogate antibody.

When the strategy for overcoming a tolerability issue is a therapeutic treatment, this may be done by administering a therapeutic agent to the second mouse, or second group of mice, in conjunction with the intravenous or intraperitoneal administration of the therapeutic or surrogate antibody to the mouse, or group of mice. In this context, in conjunction means essentially at the same time or shortly after. The second mouse, or second group of mice, is observed during a period following immediately after the administration of the therapeutic or surrogate antibody The results for the second mouse, or second group of mice, are compared to the results for the control mouse, or control group of mice, which has not received the therapeutic agent. A decreased display of the macroscopic symptoms for the second mouse, or the second group of mice, compared to the control mouse, or control group of mice, or no display of the macroscopic symptoms at all for the second mouse, or second group of mice, during the period is an indication that administration of the therapeutic agent can be used to prevent or mitigate the tolerability issue that would be associated with intravenous administration of the therapeutic antibody molecule to a human.

The therapeutic agent tested this way may be any agent or drug that is known to reverse or manage adverse events. Immune modulatory agents, such as antibodies, for example an anti-IL-6 antibody, known for use against cytokine release syndrome (Frey NV, Porter DL. Cytokine release syndrome with novel therapeutics for acute lymphoblastic leukemia. Hematology Am Soc Hematol Educ Program. 2016;2016:567-572), or immune suppressive and/or anti-inflammatory agents, such as corticosteroids or anti-histamine.

When the strategy for overcoming a tolerability issue is a different route of administration, the therapeutic or surrogate antibody is administered to the second mouse, or second group of mice, by a route of administration other than intravenous or intraperitoneal administration. The second mouse, or second group of mice, is observed during a period following immediately after the administration of the therapeutic or surrogate antibody The results for the second mouse, or second group of mice, are compared to the results for the control mouse, or control group of mice, which has received therapeutic or surrogate antibody by intravenous or intraperitoneal administration. A decreased display of the macroscopic symptoms for the second mouse, or the second group of mice, compared to the control mouse, or control group of mice, or no display of the macroscopic symptoms at all for the second mouse, or second group of mice, during the period is an indication that administration of the therapeutic antibody molecule to a human by a route of administration other than intravenous or intraperitoneal can be used to prevent or mitigate the tolerability issue that would be associated with intravenous administration of the therapeutic antibody molecule to a human.

The administration route tested this way may be any route that is known to the skilled person and that is suitable for administration of the therapeutic antibody molecule to humans and also workable for administration of the therapeutic or surrogate antibody to mice.

In some embodiments, the different route of administration, i.e. the route of administration other than intravenous or intraperitoneal administration, is subcutaneous administration. The therapeutic or surrogate antibody should then be prepared or formulated for subcutaneous administration to the second mouse, or second group of mice.

When the strategy for overcoming a tolerability issue is using a modified format of the therapeutic or surrogate antibody, this modified format of the therapeutic or surrogate antibody is administered intravenous or intraperitoneal to the second mouse. The second mouse, or second group of mice, is observed during a period following immediately after the administration of the modified therapeutic or surrogate antibody The results for the second mouse, or second group of mice, are compared to the results for the control mouse, or control group of mice, which has received the unmodified therapeutic or surrogate antibody by intravenous or intraperitoneal administration. A decreased display of the macroscopic symptoms for the second mouse, or the second group of mice, compared to the control mouse, or control group of mice, or no display of the macroscopic symptoms at all for the second mouse, or second group of mice, during the period is an indication that administration of the modified therapeutic antibody molecule to a human can be used to prevent or mitigate the tolerability issue that would be associated with intravenous administration of the therapeutic antibody molecule to a human.

The modification of the therapeutic antibody molecule tested this way may be any modification known or unknown to give rise to less or less severe toxic events in humans. For example, if a therapeutic antibody molecule that is found to be associated with a tolerability issue in connection with intravenous administration to a human is an antibody that engages Fc receptors, such a modification may be to alter the antibody so that it does not engage Fc receptors or so that it has impaired or abrogated FcγR binding compared to the therapeutic non-modified antibody, while the Fv variable sequence of the modified antibody remains the same as the therapeutic antibody molecule. As mentioned above, the Fc of the surrogate antibody should be chosen to match the therapeutic antibody molecule's Fc with respect to binding/non-binding (or engagement/non- engagement) of FcγR-binding and function.

In some embodiments, the modification is one that leads to increased engagement of Fc receptors.

It is possible to test more than one of the above strategies, or several variants of one or more than one of the above strategies, at the same time by including further mice, or groups of mice, which one mouse, or group of mice for each strategy or each variant of a strategy.

Described herein is also a corticosteroid for use in a dosing regimen to prevent or mitigate a tolerability issue in connection with intravenous administration of a therapeutic antibody molecule to a subject, as well as a method for preventing or mitigating a tolerability issue in connection with intravenous administration of a therapeutic antibody molecule to a subject comprising a dosing regimen for administration of a corticosteroid to the subject.

The therapeutic antibody molecule may be one that has been predicted to be associated with a tolerability issue in connection with intravenous administration to a human using the predictive method described above. In addition, or alternatively, the predictive method described above may have been used to predict that pre-treatment with a corticosteroid in combination with administration of the therapeutic antibody molecule to a human is likely to prevent or mitigate the tolerability issue that otherwise would be associated with intravenous administration of the therapeutic antibody molecule to a human.

The dosing regimen comprises administration of the corticosteroid to a subject in at least two doses prior to intravenous administration of the therapeutic antibody molecule. One dose ("the first dose") of the corticosteroid is administered 10-48 hours prior to start of the administration of therapeutic antibody molecule and one dose ("the second dose")of the corticosteroid is administered 5 minutes - 5 hours prior to the start of administration of the therapeutic antibody molecule .In addition to these two doses, it is possible to use further doses, such as one dose prior to "the first dose", and/or one dose between "the first dose" and "the second dose". Often, a patient is given several administrations of a therapeutic antibody molecule during a whole therapy. The two doses of corticosteroid may then be administered to the patient in connection with one or several administrations of the therapeutic antibody molecule. Preferably, the two doses are given to the patient in connection with each administration of the therapeutic antibody molecule.

In some cases, the first dose of corticosteroid is given 6-36 hours prior to start of administration of the therapeutic antibody molecule and the second dose of corticosteroid is given immediately prior to start of administration of the therapeutic antibody molecule. In this context, immediately prior means approximately 15-120 minutes prior to start of administration of the therapeutic antibody molecule.

In some cases, the first dose of corticosteroid is given 8-30 hours prior to start of administration of the therapeutic antibody molecule.

In some cases, the first dose of corticosteroid is given 16-24 hours prior to start of administration of the therapeutic antibody molecule.

In some cases, the second dose of corticosteroid is given 30-60 minutes prior to start of administration of the therapeutic antibody molecule.

In some cases, the first dose of corticosteroid is given 16-24 hours prior to start of administration of the therapeutic antibody molecule and the second dose of corticosteroid is given 30-60 minutes prior to start of administration of the therapeutic antibody molecule.

In some case, the dosing regimen comprises administration of at least two doses of the corticosteroid prior to each infusion of the antibody during the course of antibody therapy.

The corticosteroid used is preferably a potent corticosteroid, and more preferably a corticosteroid with as high potency as possible or as available. Examples of such corticosteroids are dexamethasone and betamethasone. It is possible to use either dexamethasone or betamethasone, or a a combination of dexamethasone and betamethasone.

In some cases when dexamethasone is used, the first dose is 4-20 mg

In some cases when dexamethasone is used, the second dose is 4-25 mg.

In some cases when dexamethasone is used, the first dose is 4-20 mg and second dose is 4-25 mg.

In some cases when dexamethasone is used, the first dose is 10-12 mg.

In some cases when dexamethasone is used, the second dose is 20 mg.

In some cases when dexamethasone is used, the first dose is 10-12 mg and the second dose is 20 mg.

In some cases when betamethasone is used, the first dose is 3.2-16 mg.

In some cases when betamethasone is used, the second dose is 3.2-20 mg.

In some cases when betamethasone is used, the first dose is 3.2-16 mg and the second dose is 3.2-20 mg.

In some cases when betamethasone is used, the first dose is 8-9.6 mg.

In some cases when betamethasone is used, the second dose is 16 mg.

In some cases when betamethasone is used, the first dose is 8-9.6 mg and the second dose is 16 mg.

In some cases, the dosing regimen comprises administration of an antihistamine in addition to the at least two administrations of a corticosteroid. In some cases, the anti-histamine is administered 10 minutes - 24 hours prior to start of administration of the therapeutic antibody molecule. In some cases, the antihistamine is administered 30-60 minutes prior to start of administration of the therapeutic antibody molecule.

The therapeutic antibody molecule, for with the corticosteroid is used to prevent or mitigate a tolerability issue in connection with intravenous administration is in some cases an Fc receptor binding antibody. In some cases, it is an anti-FcγRIIB antibody. In some cases, it is the anti-FcγRIIB antibody is the antibody having a light chain with SEQ ID No: 1 and a heavy chain with SEQ ID No: 2.

Described herein is also a therapeutic antibody molecule for use in the treatment of cancer, wherein the therapeutic antibody molecule is formulated for subcutaneous administration in order to prevent or mitigate a tolerability issue that would occur in connection with intravenous administration of the therapeutic antibody molecule to a subject, as well as a method for treatment of cancer comprising subcutaneous administration of a therapeutic antibody instead of intravenous administration in order to prevent or mitigate a tolerability issue.

In some cases, the therapeutic antibody molecule formulated for subcutaneous administration is an anti-FcγRIIB antibody. In some such cases, the therapeutic antibody molecule is the antibody having a light chain with SEQ ID No: 1 and a heavy chain with SEQ ID No: 2.

Described herein is also a modified format of a therapeutic antibody molecule for use in the treatment of cancer, wherein the modification of the therapeutic antibody molecule is made in order to prevent or mitigate a tolerability issue that would occur in connection with intravenous administration of the therapeutic antibody molecule to a subject, as well as a method for treatment of cancer comprising administration of such a modified format of a therapeutic antibody.

The modification of the therapeutic antibody molecule used may be any modification known to give rise to less or less severe toxic events in humans.

As mentioned above, the modification of the therapeutic antibody may also be a modification previously unknown to give rise to less or less severe toxic events in humans that has been tested with the predictive model described herein and found to be useful in order to prevent or mitigate a tolerability issue that would occur in connection with intravenous administration of the therapeutic antibody molecule to a subject.

As mentioned above, one example is that if a therapeutic antibody molecule that is found to be associated with a tolerability issue in connection with intravenous administration to a human is an antibody that engages Fc receptors, a modification used in the present context may be to alter the antibody so that it does not engage Fc receptors or so that it has impaired or abrogated FcγR binding compared to the therapeutic non-modified antibody, while the Fv variable sequence of the modified antibody remains the same as the therapeutic antibody molecule.

Thus, in some such cases, the therapeutic antibody molecule is an Fc receptor binding antibody and the modified format is an antibody having the same Fv variable sequence but having impaired or abrogated FcγR binding compared with the therapeutic antibody molecule. In some case, the therapeutic antibody is an Fc receptor binding antibody anti-FcγRIIB antibody, and in some such cases, the modified format is anti-FcγRIIB antibody is the antibody having a light chain with SEQ ID No: 1 and a heavy chain with SEQ ID No: 3.

The term subject used herein refers to a human who has been diagnosed as having a specific disease. Herein, the terms subject and patient are used interchangeably.

In some cases, the subject has been diagnosed with a cancer. In some such cases, the cancer is a B-cell malignancy. In some such cases, the cancer is selected from the group consisting of non-Hodgkin lymphoma, such as follicular lymphoma, diffuse large B cell lymphoma, mantle cell lymphoma, or chronic lymphocytic leukemia.

In some cases, the tolerability issue that is prevented or mitigated is thrombocytopenia (decrease of platelets). In some such cases, it is transient thrombocytopenia.

In some cases, the tolerability issue that is prevented or mitigated is cytokine release syndrome. In some such cases, it is transient cytokine release.

In some cases, the tolerability issue that is prevented or mitigated is elevated liver enzymes. In some such cases, it is elevated levels of aspartate aminotransferase (AST) and/or elevated levels of alanine aminotransferase (ALT).

### Additional embodiments

Embodiments of the invention is set out in the claims. Additional embodiments are the following:
1. A method for preventing or mitigating a tolerability issue in connection with intra-venous administration of a therapeutic antibody molecule to a subject comprising a corticosteroid dosing regimen,
   wherein the therapeutic antibody molecule has been predicted to be associated with a tolerability issue in connection with intravenous administration to a human using the method of any one of the claims 1-13, and/or wherein pre-treatment with the corticosteroid combination with administration of the therapeutic antibody molecule to a human has been predicted to prevent or mitigate the tolerability issue that otherwise would be associated with intravenous administration of the therapeutic antibody molecule to a human using the method of any one of the claims 5-7, or any one of the claims 10-13 when referring to any one of the claims 5-7-,
   and wherein the dosing regimen comprises administration of the corticosteroid to the subject in at least two doses prior to intravenous administration of the therapeutic antibody molecule, wherein one dose of the corticosteroid is administered 10-48 hours prior to start of the administration of therapeutic antibody molecule ("the first dose") and one dose of the corticosteroid is administered 5 minutes - 5 hours prior to the start of administration of the therapeutic antibody molecule ("the second dose").
2. A method according to embodiment 1, wherein the first dose is given 6-36 hours prior to start of administration of the therapeutic antibody molecule and the second dose is given 15-120 minutes prior to start of administration of the therapeutic antibody molecule.
3. A method according to embodiment 1 or 2, wherein the first dose is given 16-24 hours prior to start of administration of the therapeutic antibody molecule.
4. A method according to any one of the embodiments 1-3, wherein the second dose is given 30-60 minutes prior to start of administration of the therapeutic antibody molecule.
5. A method according to any one of the embodiments 1-4, wherein the dosing regimen comprises administration of the at least two doses of the corticosteroid prior to each infusion of the antibody during the course of antibody therapy.
6. A method according to any one of the embodiments 1-5, wherein the corticosteroid is dexamethasone or betamethasone or a combination of dexamethasone and betamethasone.
7. A method according to any one of the embodiments 1-6, wherein the corticosteroid is dexamethasone and wherein the first dose is 4-20 mg and the second dose is 4-25 mg.
8. A method according to embodiment 7, wherein the first dose is 10-12 mg and the second dose is 20 mg.
9. A method according to any one of the embodiments 1-6, wherein the corticosteroid is betamethasone and wherein the first dose is 3.2-16 mg and the second dose is 3.2-20 mg.
10. A method according to embodiment 9, wherein the first dose is 8-9.6 mg and the second dose is 16 mg.
11. A method according to claim any one of the embodiments 1-10, wherein the dosing regimen further comprises administration of an antihistamine 10 minutes - 24 hours prior to start of administration of the therapeutic antibody molecule.
12. A method according to any one of the embodiments 1-11, wherein the antibody is an Fc receptor binding antibody.
13. A method according to any one of the embodiments 1-12, wherein the antibody is an anti-FcγRIIB antibody.
14. A method according to embodiment 13, wherein the anti-FcγRIIB antibody is the antibody having a light chain with SEQ ID No: 1 and a heavy chain with SEQ ID No: 2.
15. A method for treatment of cancer comprising subcutaneous administration of a therapeutically active amount of a therapeutic antibody molecule which has been predicted to be associated with a tolerability issue in connection with intravenous administration to a human using the method of any one of the claims 1-13 and/or wherein the subcutaneous route of administration of therapeutic antibody molecule to a human has been predicted to prevent or mitigate the tolerability issue that otherwise would be associated with intravenous administration of the therapeutic antibody molecule to a human using the method of claim 8, or any one of the claims 10-13 when referring to claim 8.
16. A method according to embodiment 15, wherein the antibody is an anti-FcγRIIB antibody.
17. A method according to embodiment 16, wherein the anti-FcγRIIB antibody is the antibody having a light chain with SEQ ID No: 1 and a heavy chain with SEQ ID No: 2.
18. A method for treatment of cancer comprising administration of a therapeutically active amount of a modified format of a therapeutic antibody, wherein the therapeutic antibody molecule has been predicted to be associated with a tolerability issue in connection with intravenous administration to a human using the method of any one of the claims 1-13 and/or wherein administration of the therapeutic antibody molecule in the modified format to a human has been predicted to prevent or mitigate the tolerability issue that otherwise would be associated with intravenous administration of the therapeutic antibody molecule to a human using the method of claim 9, or any one of the claims 10-13 when referring to claim 9,
   and wherein the therapeutic antibody molecule is an Fc receptor binding antibody and the modified format is an antibody having the same Fv variable sequence but having impaired or abrogated FcγR binding compared with the therapeutic antibody molecule.
19. A method according to embodiment 18, wherein the antibody is an anti-FcγRIIB antibody.
20. A method according to embodiment 19, wherein the anti-FcγRIIB antibody is the antibody having a light chain with SEQ ID No: 1 and a heavy chain with SEQ ID No: 2 with a N297Q mutation in the heavy chain.

### BRIEF DESCRIPTION OF THE DRAWINGS

In the examples below, reference is made to the following figures:

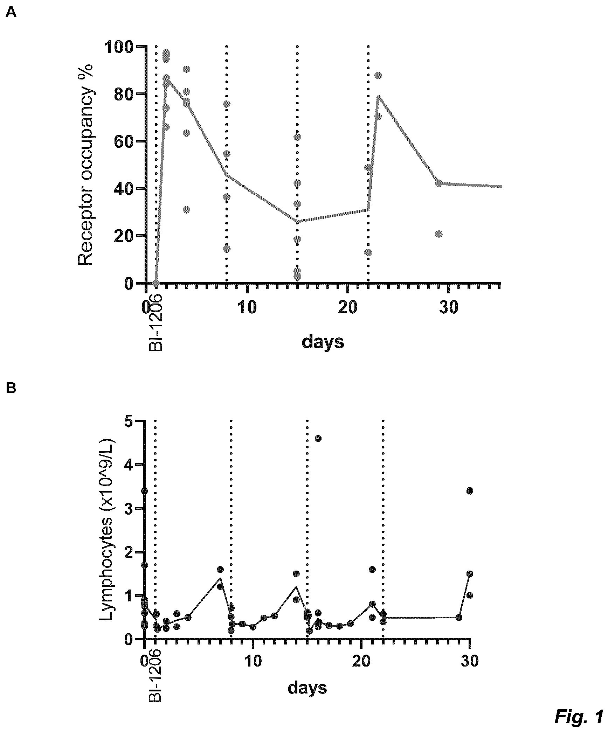

Figure 1. Transient FcγRIIB receptor occupancy and peripheral lymphocyte depletion after BI-1206 infusion. Fig. 1 A) FcγRIIB receptor occupancy on peripheral B cells after BI-1206 infusion followed over time in subjects having received 100 mg BI-1206 (n=8). Vertical dashed lines denote BI-1206 infusions. At second and third BI-1206 infusion receptor occupancy data is only available pre-infusion. Line denotes the median. Based on preliminary data. Fig. 1 B) Peripheral lymphocytes after BI-1206 infusion followed over time in subjects having received 100 mg BI-1206 (n=9). Vertical dashed lines denote BI-1206 infusions. Line denotes the median. Based on preliminary data.

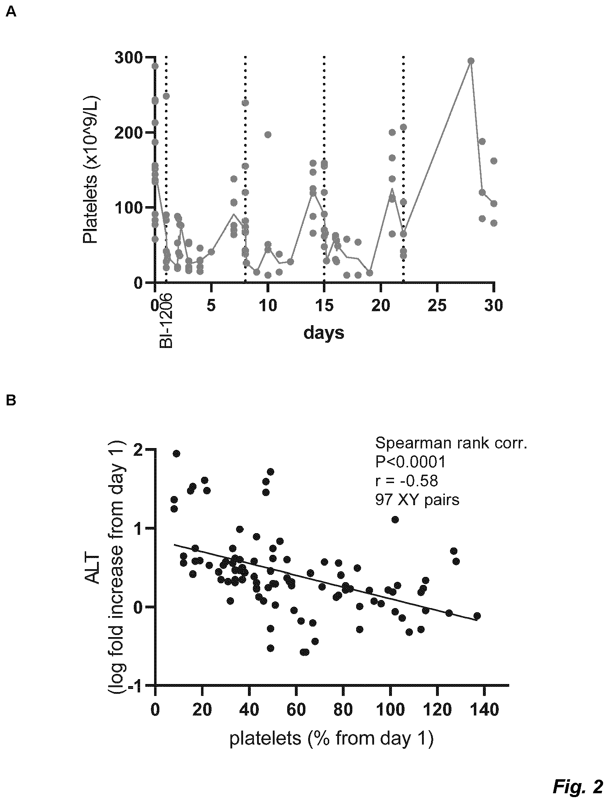

Figure 1. Transient decrease of platelets associates with increase in ALT after BI-1206 infusion. Fig. 2 A) Platelet count after BI-1206 infusion followed overtime in subjects having received 70-100 mg BI-1206 (n=16). Vertical dashed lines denote BI-1206 infusions. Line denotes the median. Based on preliminary data. Fig. 2 B) Fold increase of ALT versus percent platelet depletion (n=16). Changes are calculated in relation to day 1, pre-BI-1206-infusion. Based on preliminary data.
Figure 2. Cytokine release after BI-1206 infusion. Number of patients with plasma/serum increase of different cytokines detected at end of BI-1206 infusion. To be considered as positive the value should be > 10-fold increased as compared to pre-infusion and >10-fold above the upper limit of normal range (ULN). Samples for analysis have been available from 5 subjects receiving ≥70 mg BI-1206. Based on preliminary data.

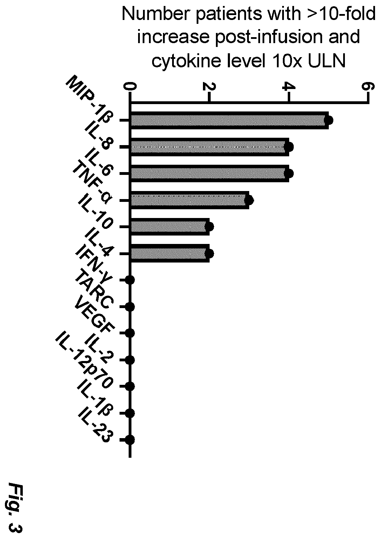

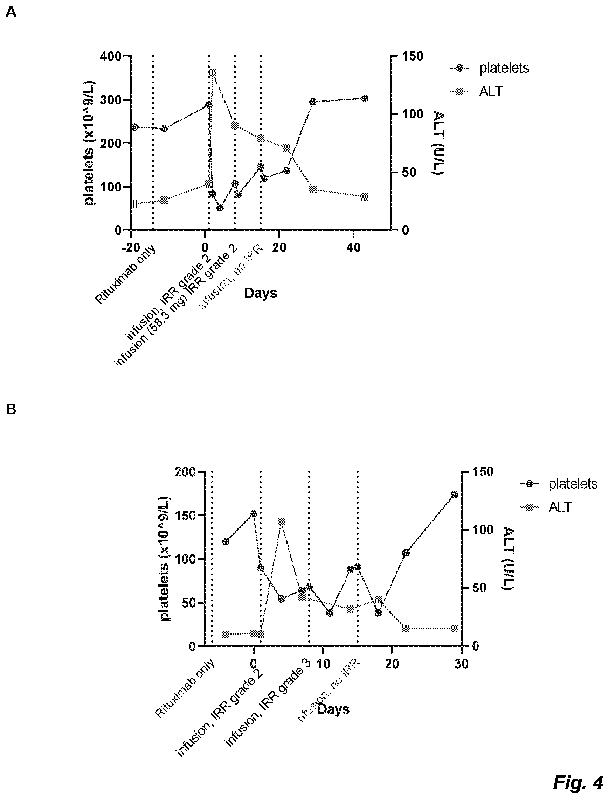

Figure 3. Two doses of dexamethasone relieved IRRs, platelet decrease, and transaminase increase in two subjects receiving BI-1206. Platelet count and ALT in subjects 501-001 (Fig. 4 A) and 503-002 (Fig. 4 B). Vertical dashed lines denote antibody infusions. The first infusion in each subject was rituximab alone and the following BI-1206 and rituximab. Grade of IRR at each antibody infusion is indicated. 501-001 and 503-002 received 12 mg and 4 mg dexamethasone, respectively, the evening before and again 20 mg dexamethasone 30 minutes prior to the third administration of BI-1206 (70 mg) during induction therapy. None of the subjects suffered any IRRs after this premedication regimen with two doses of dexamethasone. During the previous two infusions with BI-1206, where dexamethasone (20 mg) was given only 30 minutes prior to infusion, IRRs (grade 2-3) had been experienced. In addition, in subject 501-001 and 503-002 no/low platelet decrease, and no ALT/AST increase was seen after BI-1206 administration when premedicating with two doses of dexamethasone.
Figure 4. Macroscopic symptoms after injection of murine surrogate anti-CD32b (AT-130-2 IgG2a). The murine surrogate anti-CD32b (AT-130-2 IgG2a) was injected into wildtype C57/BL6 mice through 3 different injection routes, intravenously i.v., intraperitoneally i.p. or subcutaneously s.c. Macroscopic symptoms were seen after i.v. and i.p. injection. These macroscopic symptoms included isolation, decreased activity, impaired balance, piloerection, hunching followed by un-natural body posture and the macroscopic symptoms were scored from 0-2 based on the observations. When titrating the i.v. dose a rapid onset of IRRs was seen 5-7 minutes post injection. The same timing and severity of macroscopic symptoms was seen down to 10 µg (0.5 mg/kg). However, at 1 µg (0.05 mg/kg) no macroscopic symptoms were seen. When administrating 200 µg (10 mg/kg) i.p. a delay in onset of macroscopic symptoms was seen in comparison with i.v. injection with the macroscopic symptoms appearing 20-30 minutes post injection. In contrast to the i.v. injection route all mice in this group did not display macroscopic symptoms and the macroscopic symptoms were less severe in several mice. When increasing the i.p. dose to 400 µg (20 mg/kg) the onset of macroscopic symptoms was still delayed compared to the i.v. injection route however, all mice displayed macroscopic symptoms to the same extent and grade as 200 µg i.v. All mice had fully recovered 1 hour post injection. Finally, when administrating 200 µg to mice s.c., no macroscopic symptoms were seen (up to 24 hours post injection). When increasing the s.c. dose to 400 µg the mice remained unaffected.

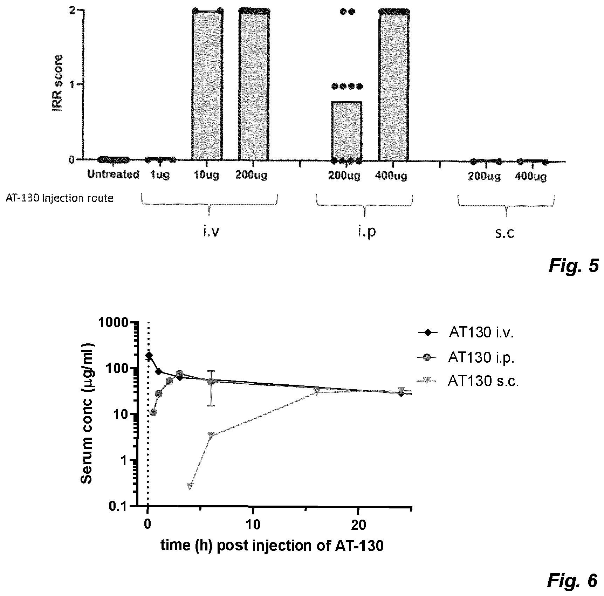

Figure 5. Pharmacokinetic profiles of AT-130-2 IgG2a in C57BL/6 mice. Observed AT-130-2 serum concentrations in mice treated with AT-130 via three different administrations routes; 200 µg AT-130-2 via i.v. injection, 200 µg AT-130-2 via i.p. injection and 400 µg AT-130-2 via s.c. injection. Presented data are the mean of 1-4 mice/dose groups. Abbreviations: h=hour(s); i.p. intraperitoneal, i.v. intravenous, s.c. subcutaneous.
Figure 6. Correlation between macroscopic symptoms (denoted IRRs in this figure) and high, rapid exposure of AT-130-2 rather than time of FcγRIIB saturation. The serum concentration of AT-130-2 IgG2a (line with dots) is plotted against the grade of macroscopic symptoms (line with squares) for the different administration routes (A: i.v., B: i.p. and C: s.c.). When comparing the serum concentration of AT-130-2 and the presumed receptor occupancy (RO) (dotted line, 10 mg/ml gives 100% receptor saturation) with the onset, severity and duration of IRRs it is clear that there is a correlation between high and rapid exposure of AT-130-2, rather than time of FcγRIIB saturation. Tolerability showing a clear pattern of s.c. > i.p. > i.v. with RO being sustained for a long period of time post recovery from macroscopic symptoms.

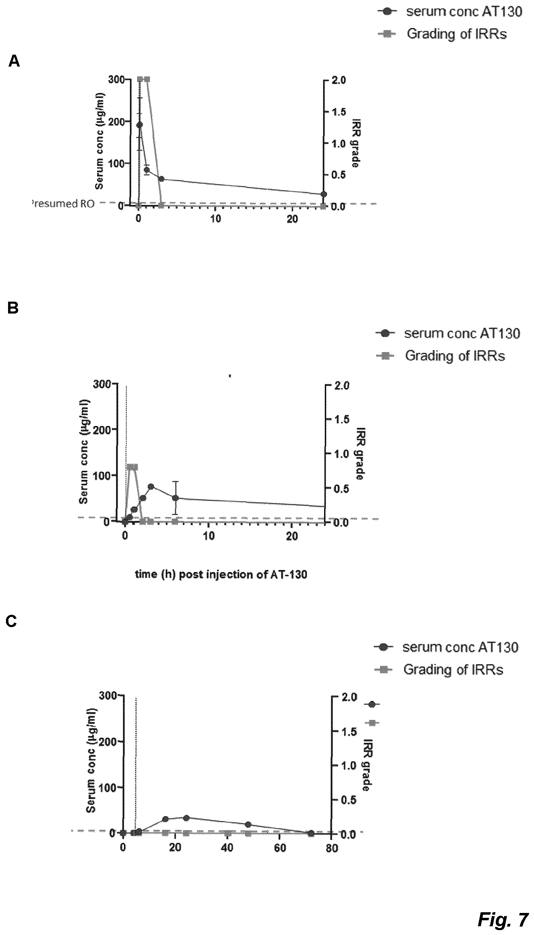

Figure 7. Timing of platelet (PLT) nadir is correlated to the route of administration and time to FcγRIIB saturation. Mice were bled at different time points post injection of AT-130-2 IgG2a and the blood was analyzed for blood platelet count (PLT) in an automated Vetcount. A nadir in platelet count was seen at the same time as onset of macroscopic symptoms after injection of AT-130-2 through both the i.v. and i.p. administration route. For the s.c. administration route where no macroscopic symptoms are seen, a moderate drop was seen 10 hours post injection correlating with the time to FcγRIIB saturation according to PK. In all cases the PLT decrease was transient and was restored to values in the normal range within 8 hours post injection. Mice presenting macroscopic symptoms are shown with filled bars.

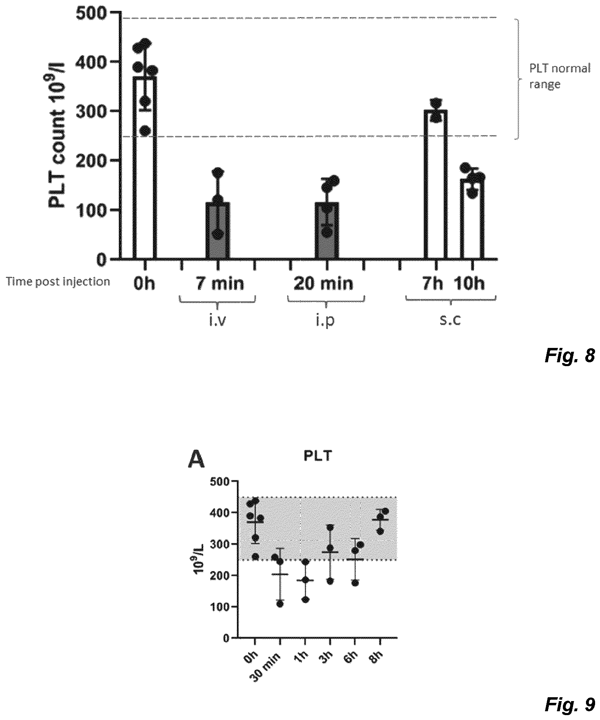

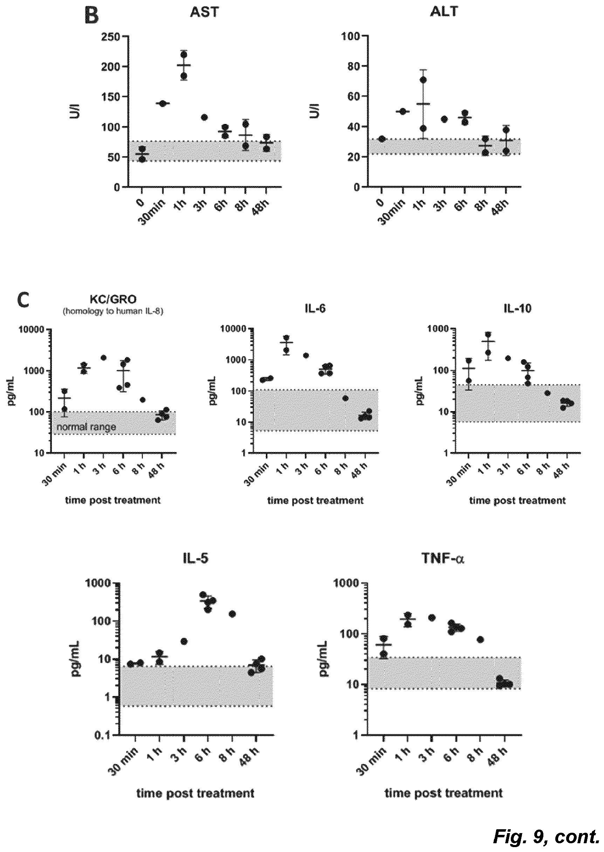

Figure 8: Transient platelet decrease, transaminase increase and cytokine release, after administration of AT-130-2 IgG2a. Mice were bled at different time points after i.p. injection of 200 µg AT-130-2 and the blood was analyzed for platelet counts (Fig. 9 A), transaminases (Fig. 9 B) and cytokines (Fig. 9 C). A transient PLT decrease was seen with the PLT counts fully restored 8 hours post injection (Fig. 9 A). An increase in transaminases (AST and ALT) with a peak 1h post injection was the only clinical chemistry parameter affected by AT-130-2 injection (Fig. 9 B). These increases were just like the PLT decrease transient. The same transient increase was seen when AT-130-2 was injected i.v. and no increase in transaminases was detected when AT-130-2 was injected s.c. (data not shown). A panel of cytokines including the analytes IFN-γ, IL-1β, IL-2, IL-4, IL-5, IL-6, IL-10, IL-12p70, KC/GRO, TNF-α were analyzed at different time points post injection of 200 mg i.p. Of the analyzed cytokines IL-5, IL-6, IL-10, KC/GRO, TNF-α showed a transient increase with them all except for IL-5 peaking 1-3 hours post injection (Fig. 9 C). IL-5 showed a delayed peak 3-8 hours post injection (Fig. 9 C). These are the same cytokines that have been indicated to increase in some patients in the clinical studies with BI-1206.

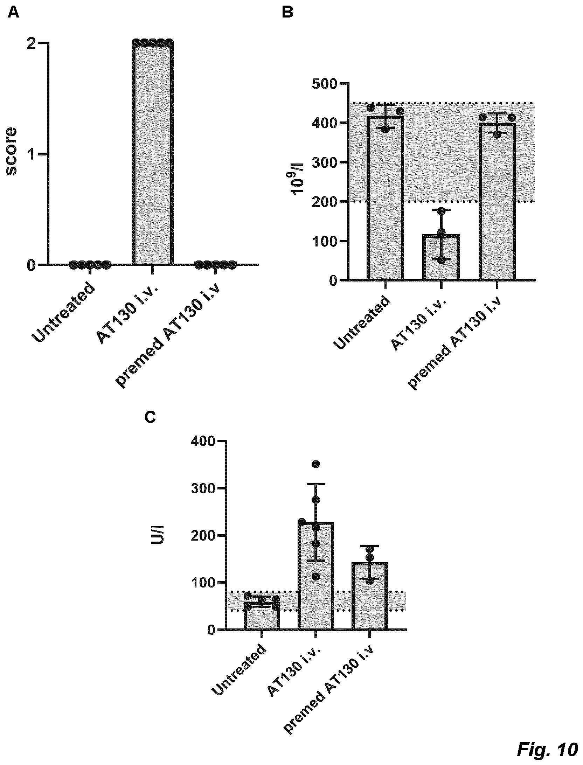

Figure 9. Premedication with 2 doses of corticosteroids inhibits macroscopic symptoms associated with AT-130-2 IgG2a administration. Mice were either pretreated with 40 mg/kg betamethasone 24 hours and 1 hour or left untreated pre i.v. injection of 10 mg/kg AT-130-2 IgG2a. Mice were observed for macroscopic symptoms (Fig. 10 A) and bleed 20 minutes post injection. The blood was analyzed for (Fig. 10 B) platelet counts, (Fig. 10 C) transaminases and cytokines. Premedication with betamethasone completely inhibited the macroscopic symptoms (Fig. 10 A), decrease in platelet count (Fig. 10 B) and reduced the transaminases increase (Fig. 10 C). The transient cytokine release seen after i.v. injection of AT-130-2 was also inhibited by the premedication (data not shown). The same results were seen when using dexamethasone (data not shown).

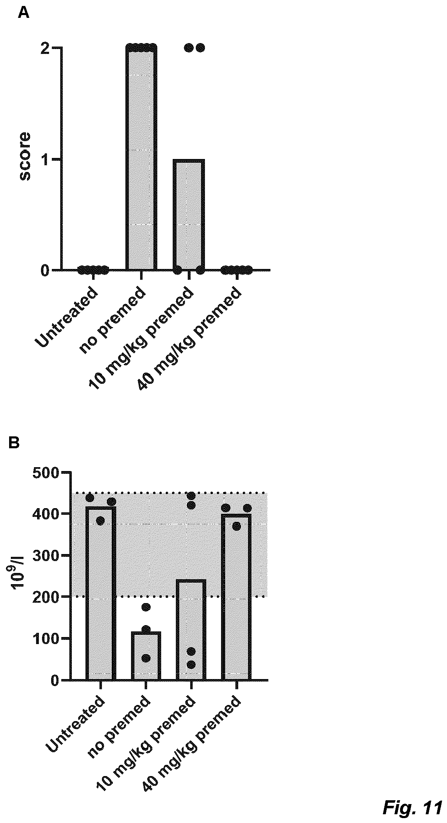

Figure 10. The dose of premedication is of importance. Mice were either pretreated with 40 mg/kg or 10 mg/kg Betamethasone 24 hours and 1 hour (premed) pre i.v. injection of 10 mg/kg AT-130-2 IgG2a. Mice were observed for macroscopic symptoms (Fig. 11 A) and bleed 20 minutes post injection. The blood was analyzed for (Fig. 11 B) platelet counts. Premedication with 10 mg/kg Betamethasone did not completely inhibited macroscopic symptoms (Fig. 11 A) or decrease in platelet count (Fig. 11 B) indicating that lowering the dose of premedication might reduce its potential to inhibit macroscopic symptoms and platelet decreases.

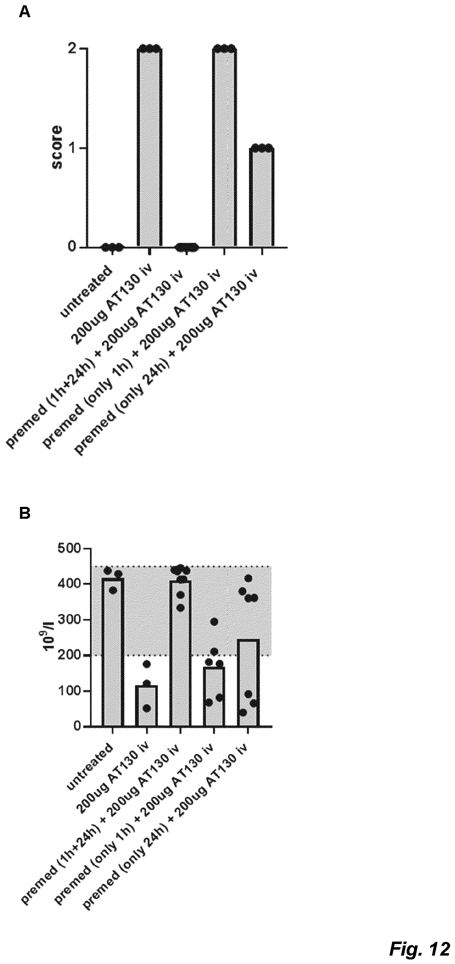

Figure 11. Steroid premedication 1 hour prior to infusion is not sufficient to inhibit IRRs. Mice were either pretreated with 40 mg/kg Betamethasone 24 hours, 1 hour or 24 hours + 1 hour pre i.v. injection of 10 mg/kg AT-130-2 IgG2a. Mice were observed for macroscopic symptoms (Fig. 12 A) and bleed 10-20 minutes post injection. The blood was analyzed for platelet counts (Fig. 12 B). Single premedication with betamethasone 1 hour post injection of AT-130-2 did not inhibited macroscopic symptoms (Fig. 12 A) or platelet decrease (Fig. 12 B) and single premedication with betamethasone 24 hours post injection of AT-130-2 did only reduce the macroscopic symptoms to a grade 1. This indicates that two doses of steroid treatment are needed to completely inhibit tolerability issues.

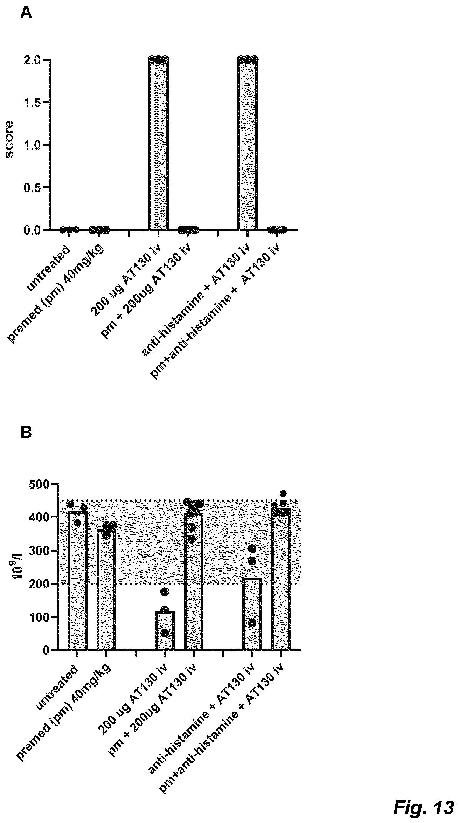

Figure 12. Premedication with anti-histamine is not enough to inhibit tolerability issues associated with AT-130-2 IgG2a administration. Mice were either pretreated with anti-histamine alone or with 40 mg/kg Betamethasone (24 hours and 1 hour) +/- anti-histamine pre i.v. injection of 10 mg/kg AT-130-2 IgG2a. Mice were observed for macroscopic symptoms (Fig. 13 A) and bleed approximately 20 minutes post injection. The blood was analyzed for platelet counts (Fig. 13 B). Premedication with anti-histamine alone did not inhibit macroscopic symptoms (Fig. 13 A) but did seem to improve decrease in platelet counts (Fig. 13 B). The addition of anti-histamine to 40 mg/kg Betamethasone (24 hours and 1 hour) pre i.v. injection of 10 mg/kg AT-130-2 IgG2a did not affect macroscopic symptoms or platelet counts. The same results were seen when using three different types of anti-histamines (Zyrlex, Zantac or Au, data not shown).

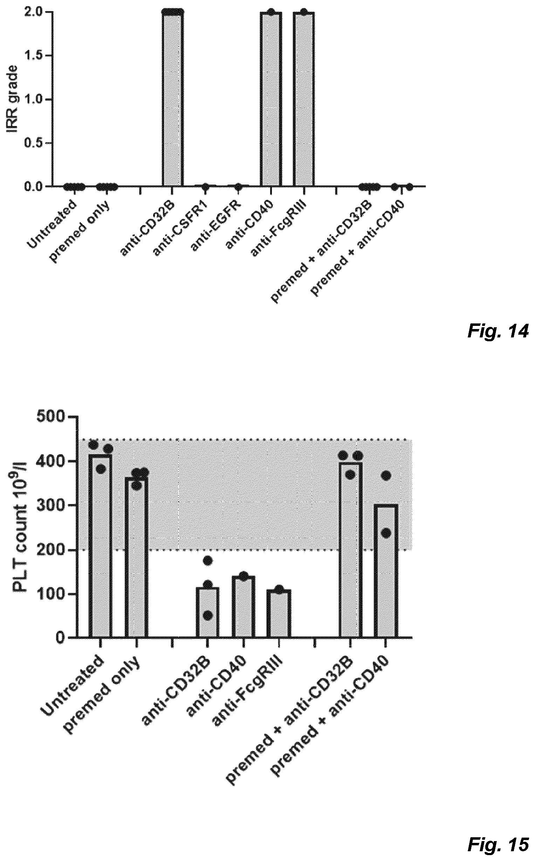

Figure 14 shows that several but not all murine surrogate antibodies induce IRRs. The murine surrogates anti-CD32b antibody (AT-130-2 mlgG2a), anti-CSFR1 (AFS98 rlgG2a), anti-EGFR (7A7 mlgG2a), anti-CD40 (FGK4.5 rlgG2a) and anti-FcγRIII (AT154-2 mlgG2a) were injected into wildtype C57/BL6 mice intravenously i.v. IRRs was seen after injection of anti-CD32b, anti-CD40 and anti-FcγRIII. The IRRs included isolation, decreased activity, impaired balance, piloerection, hunching followed by un-natural body posture and the IRRs were scored from 0-2 based on the observations. No IRRs were seen for anti-EGFR or anti-CFSR1. When mice were pretreated with 40 mg/kg betamethasone 24h and 1h pre-injection of anti-CD32b or anti-CD40 no IRRs were seen (anti-FcγRIII was not evaluated with premedication). Indicating that premedication can inhibit IRRs related to different antibodies and targets.
Figure 15 shows that antibodies inducing IRRs also induce platelet decrease. The murine surrogates anti-CD32b antibody (AT-130-2 mlgG2a), anti-CD40 (FGK4.5 rlgG2a) and anti-FcγRIII (AT154-2 mlgG2a) were injected into wildtype C57/BL6 mice intravenously i.v. Platelet counts were analyzed in fresh blood 20 min post injection using a Vetscan (Vetscan HM5 Abaxis, Triolab). A decrease in platelet counts was seen after injection of anti-CD32b, anti-CD40 and anti-FcγRIII. When mice were pretreated with 40 mg/kg betamethasone 24h and 1h pre-injection of anti-CD32b or anti-CD40 no platelet decrease were seen (anti-FcγRIII was not evaluated with premedication). Indicating that premedication can inhibit platelet decrease related to different antibodies and targets.

### EXAMPLES

Specific, non-limiting examples which embody certain aspects of the invention will now be described. These examples should be read together with the brief description of the drawings provided above.

In these examples, an antibody denoted BI-1206 is used. This antibody has the following light and heavy chains:
Light chain: 
QSVLTQPPSASGTPGQRVTISCTGSSSNIGAGYDVHWYQQLPGTAPKLLIYADDHRP-
SGVPDRFSGSKSGTSASLAISGLRSEDEADYYCASWDDSQRAVIFGGGTKLTVLGQP-
KAAPSVTLFPPSSEELQANKATLVCLISDFYPGAVTVAWKADSSPVKAGVETTTPSKQ-
SNNKYAASSYLSLTPEQWKSHRSYSCQVTHEGSTVEKTVAPTECS (SEQ. ID. No: 1)
Heavy chain:

EVQLLESGGGLVQPGGSLRLSCAASGFTFSSYGMHWVRQAPGKGLEWMAVISYDG-
SNKYYADSVKGRFTISRDNSKNTLYLQMNSLRAEDTAVYYCARELYDAFDIWGQGTL-
VTVSSASTKGPSVFPLAPSSKSTSGGTAALGCLVKDYFPEPVTVSWNSGALTSGVHTF-
PAVLQSSGLYSLSSVVTVPSSSLGTQTYICNVNHKPSNTKVDKKVEPKSCDKTHTCPP-
CPAPELLGGPSVFLFPPKPKDTLMISRTPEVTCVVVDVSHEDPEVKFNWYVDGVEVH-
NAKTKPREEQYNSTYRVVSVLTVLHQDWLNGKEYKCKVSNKALPAPIEKTISKAKGQP-

REPQVYTLPPSRDELTKNQVSLTCLVKGFYPSDIAVEWESNGQPENNYKTTPPVLDSD-
GSFFLYSKLTVDKSRWQQGNVFSCSVMHEALHNHYTQKSLSLSPGK (SEQ. ID. No: 2)

A modified format of BI-1206 is format wherein the glycosylation site at N297 (marked in bold above) is mutated to a Q (marked in bold below), i.e. an N297Q mutation, resulting in the following heavy chain:

EVQLLESGGGLVQPGGSLRLSCAASGFTFSSYGMHWVRQAPGKGLEWMAVISYDG-
SNKYYADSVKGRFTISRDNSKNTLYLQMNSLRAEDTAVYYCARELYDAFDIWGQGTL-
VTVSSASTKGPSVFPLAPSSKSTSGGTAALGCLVKDYFPEPVTVSWNSGALTSGVHTF-
PAVLQSSGLYSLSSVVTVPSSSLGTQTYICNVNHKPSNTKVDKKVEPKSCDKTHTCPP-
CPAPELLGGPSVFLFPPKPKDTLMISRTPEVTCVVVDVSHEDPEVKFNWYVDGVEVH-
NAKTKPREEQYQSTYRVVSVLTVLHQDWLNGKEYKCKVSNKALPAPIEKTISKAKGQP-
REPQVYTLPPSRDELTKNQVSLTCLVKGFYPSDIAVEWESNGQPENNYKTTPPVLDSD-
GSFFLYSKLTVDKSRWQQGNVFSCSVMHEALHNHYTQKSLSLSPGK (SEQ. ID. No: 3)

As surrogate antibody and control antibody, the anti-mouse CD32B antibody AT130-2 as IgG2a isotype and the control antibody AT130-2 N297A as IgG1 isotype are used below. AT130-2 as IgG2a isotype is commercially available, for example from ThermoFisher Scientific as Catalog # 12-0321-82, however # 12-0321-82 is a PE conjugate so the antibody then should be modified so that it is not a conjugate. AT130-2 N297A as IgG1 isotype may be produced by any known method including substituting N in position 297 (as identified above) with A.

### Example 1 - Target: FcγRIIB

### Background

Biolnvent International AB has developed the therapeutic monoclonal antibody BI-1206 with anti-tumor activity that can be used as single therapy or in combination with anti-CD20 targeting therapeutics or other clinically validated checkpoints inhibitors. BI-1206 binds with high specificity to CD32B (FcγRIIB) and is currently evaluated in two clinical phase I/IIa studies, CRUKD/16/001 and 17-BI-1206-02, treating patients with chronic lymphocytic leukemia (CLL) and B-cell non-Hodgkin's lymphoma (B-cell NHL). All below described data from the clinical studies is preliminary, only partially quality controlled and should be considered as illustrative of the pharmacodynamic effects and tolerability associated with BI-1206. Part of the data is based on personal communication with individual investigators.

To date up to 100 mg of BI-1206 have been administrated, as monotherapy or in combination with rituximab, to 24 human subjects. 100 mg BI-1206 shows transient receptor saturation on peripheral B cells with 100%, or close to 100%, receptor occupancy for up to 48 hours (**Error! Reference source not found.**). Correspondingly a transient depletion of peripheral B lymphocytes is seen, recovered within approximately 7 days (**Error! Reference source not found.**). This is in line with pre-clinical in vivo models using hFcγRIIB mice where it has been demonstrated that a sustained receptor saturation is necessary to achieve sustained B lymphocyte depletion.

Frequent infusion related reactions (IRRs) have been seen during BI-1206 infusions in human subjects. Administration of ≥50 mg BI-1206 is also associated with a transient decrease in platelets (Figure 1). Thrombocytopenia has not been serious nor associated with bleeding and most episodes resolved within a week. There appears to be a connection between platelet decrease and elevated transaminases (i.e. alanine transaminase (ALT) and aspartate transaminase (AST)), where the ALT and AST increase has been significant in 3 out of 16 subjects receiving ≥70 mg BI-1206 (Figure 1).

Moreover, a transient cytokine release has been observed in 5 out 5 subjects receiving ≥70 mg BI-1206 where plasma or serum has been available for analysis. The cytokine release includes macrophage inflammatory protein (MIP)-1β, tumor necrosis factor (TNF)-α, interleukin (IL)-10, IL-8, IL-6, and IL-4, and the peak is seen immediately after infusion and cytokines are always normalized within 24 hours (Figure 2). The cytokine release has not been associated with clinical symptoms.

In clinical study 17-BI-1206-02 three subjects (501-001, 503-002 and 201-003) have shown improved tolerability when receiving two doses of corticosteroids prior to BI-1206 administration. Subjects 501-001 and 503-002 received 12 mg and 4 mg dexamethasone, respectively, the evening before and again 20 mg dexamethasone 30 minutes prior to the third administration of BI-1206 (70 mg) during induction therapy. Both subjects did not suffer IRRs after this premedication regimen using two doses of dexamethasone. During the previous two infusions with BI-1206, where dexamethasone (20 mg) was given only 30 minutes prior to infusion, IRRs (grade 2-3) had been experienced. In addition, in subject 501-001 and 503-002 no/low platelet decrease, and no ALT/AST increase was seen after BI-1206 administration when premedicating with two doses of dexamethasone (Figure 3). The third subject (201-003) had received 9 administrations of 30mg of BI-1206 (4 doses during induction phase and 5 doses during maintenance phase) and repeatedly experienced IRR's. At the 10th BI-1206 administration premedication with two doses of dexamethasone was used, and IRR's were improved to grade 1. In subject 201-003 which received a lower dose of BI-1206 (30 mg) platelet decrease or ALT/AST increase had never been seen.

### Materials & Methods

### Test and control substances

The anti-mouse CD32B IgG2a clone AT130-2 and the control antibody (AT130-2 N297A) were transiently expressed in HEK293 cells. The specificity of the purified research batches was demonstrated in a luminescence-based enzyme linked immunosorbent assay (ELISA) or in flow cytometry analyses. Endotoxin-levels of antibodies were found to be <0.1 IU/mL as determined by the LAL-Amoebocyte test.

| Antibody clone | Description |
|---|---|
| AT-130-2 IgG2a | Mouse surrogate of BI-1206 as described above |
| AT-130-2 IgG1 N297A | Fc null version of mouse surrogate of BI-1206 as described above |

### Mice

Six to eight weeks-old (17-20 g) female C57/BL6 mice were obtained from Taconic. Mice were injected either intra-venous (i.v.), intra-perinatal (i.p.) or sub-cutaneous (s.c.) with mouse anti-CD32B AT-130-2 IgG2a in doses ranging from 1 µg - 400 µg/mouse.

### Premedication

For the corticosteroid treatment, Betapred (betamethasone, VNR: 008938, Alfa-sigma S.P.A.) or Dexamethasone (Cat. No: S1322, batch no: 02, Selleckchem) was used. For the anti-histamine treatment Zyrlex (10 mg/ml, VNR: 523084, MACURE PHARMA ApS), Zantac (25 mg/ml, VNR: 077875, GlaxoSmithKline AB) or Aeurius (0.5 mg/ml, VNR: 097288, Merck Sharp & Dohme BV) was used.

### Animal monitoring

Mice were monitored post injection with regard to changes in behavior and macroscopic symptoms such as isolation, mobility, and fur condition. Macroscopic IRRs scoring system of 0-2 was set up based on the observations:

| Scoring | Macroscopic symptoms |
|---|---|
| 0 | No visible symptoms |
| 1 | Isolation, decreased activity |
| 2 | Isolation, decreased activity, impaired balance, piloerection, hunching followed by un-natural body posture |

### Blood sampling

Blood samples were collected from vena saphena for instant blood count analysis. For serum concentrations of AT130-2, liver enzyme and cytokine analysis, the mice were bled from the aorta under isoflurane anesthesia just prior to sacrifice.

### Serum concentrations of AT130-2

Serum concentrations of AT130-2 mAb has been was quantified using a sandwich ELISA. Briefly, recombinant CD32B protein (Sino Biological #50030-M08H) was used as coating. Diluted samples were added to the ELISA plate, and following incubation and washing steps, detection was conducted via an HRP conjugated polyclonal donkey-anti-mouse-IgG Ab (Jackson #715-035-151). Subsequently the Pico Chemilumines-cent Substrate (ThermoFisher #37069) were used and plate reading was performed with a Tecan Ultra Microplate reader.

### Platelet count

Platelet counts were analyzed in fresh blood using a Vetscan (Vetscan HM5 Abaxis, Triolab).

### Transaminases

Transaminases were analyzed shipping of frozen serum samples to (IDEXX Bio-Research Vet Med Labor GmbH).

### Cytokines

To study the potential contributors of infusion-related reactions (IRRs) in the mice, cytokine release has been evaluated at selected timepoints in association with i.p. injection of AT-130-2 mAb. Serum samples frozen once were thawed and diluted x2 or x4. Cytokines were analyzed with the V-plex Proinflammatory Panel 1 Mouse kit (MesoScale Discovery #K15048D), including the analytes interferon(IFN)-γ, interleukin(IL)-1β, IL-2, IL-4, IL-5, IL-6, IL-10, IL-12p70, KC/GRO, tumor necrosis factor (TNF)-α. The assay followed the manufacturer's protocol as outlined briefly: 50 µL of sample and calibration standard were added to the MSD plates and incubated. Following washing, 25 µL of SULFO-TAG detection antibody mixture were added to each well of the corresponding plate. The plates were analyzed on a QuickPlex SQ120 Reader instrument (MSD) and cytokine concentration was calculated using the MSD software (Discovery Workbench, 2013; version LSR-4-0-12).

### Results

### Macroscopic symptoms after murine surrogate anti-CD32b IgG2a (AT-130-2)

The murine surrogate anti-CD32b (AT-130-2 IgG2a) was injected into wildtype C57/BL6 mice through 3 different injection routes, intravenously (i.v.), intraperitoneally (i.p.) or subcutaneously (s.c.). At 200 µg (corresponding to 10 mg/kg) a rapid onset of infusion-related reactions (IRRs) was seen 5-7 minutes after i.v. injection. These IRRs included isolation, decreased activity, impaired balance, piloerection, hunching followed by un-natural body posture. Blood sampling of these mice indicated reduced blood pressure. 10-15 minutes post IRRs onset these mice started to recover and 1h post injection no macroscopic symptoms were seen.

When titrating the i.v. dose, the same timing and severity of macroscopic symptoms was seen down to 10 µg (0.5 mg/kg). However, at 1 µg (0.05 mg/kg) no IRRs were seen (Figure 4).

When administrating the same dose 200 µg (10mg/kg) i.p. a delay in IRRs onset was seen with the IRRs appearing 20-30 minutes post injection. In contrast to the i.v. injection route all mice in this group did not display IRRs and the IRRs were less severe in several mice (Figure 4)

When increasing the i.p. dose to 400 µg (20mg/kg) the onset of IRRs was still delayed compared to the i.v. injection route however, all mice displayed IRRs to the same extent and grade as 200 µg i.v. (Figure 4). All mice had fully recovered 1h post injection.

Finally, when administrating 200 µg to mice s.c. no IRRs were seen (up to 24h post injection). When increasing the s.c. dose to 400 µg the mice remained unaffected Figure 4.

When administrating the Fc-null version of AT-130-2, AT-130-2 IgG1 N297A i.v. no IRRs were seen, indicating that Fc-binding is necessary to incite the symptoms associated with AT-130-2.

The pharmacokinetic profiles of AT-130-2 was assessed for i.v., i.p., and s.c. injection Figure 5.

When comparing the PK and the presumed receptor occupancy (RO, based on separate experiments not shown here where it was shown that 10 µg/ml gives 100% receptor saturation) with the onset, severity and duration of IRRs it is clear that there is a correlation between high and rapid exposure of AT-130-2, rather than time of FcyRIIB saturation. Tolerability showing a clear pattern of s.c. > i.p. > i.v. with RO being sustained for a long period of time post IRRs recovery Figure 6.

### Platelets, transaminases and cytokines

To investigate if the IRRs seen in these mice were associated with other parameters seen in the clinical studies with BI-1206 mice were bled at the onset of IRRs and the blood was analyzed for blood cell count, clinical chemistry parameters and cytokines. In the case of s.c. injection where no IRRs occurred, mice were bled at different timepoints post injection. A decrease in platelet count (PLT) was seen at the same time as IRRs onset after injection of AT-130-2 through both the i.v. and i.p. administration route (Figure 7). For the s.c. administration route only a moderate drop was seen 10h post injection (Figure 7). In all cases the PLT decrease was transient and was restored to values in the normal range within 8h post injection (data for 200 µg AT-130-2 injected i.p. is shown in Figure 8A).

With regard to clinical chemistry parameters an increase in transaminases (AST and ALT) with a peak 1h post injection was the only parameter affected by AT-130-2 injection. These increases were just like the PLT decrease transient (Figure 8B). The same transient increase was seen when AT-130-2 was injected i.v. and no increase in transaminases was detected when AT-130-2 was injected s.c.

A panel of cytokines including the analytes IFN-γ, IL-1β, IL-2, IL-4, IL-5, IL-6, IL-10, IL-12p70, KC/GRO, TNF-α were analyzed at different time points post injection of 200 µg i.p. Of all the analyzed cytokines IL-5, IL-6, IL-10, KC/GRO, TNF-α showed a transient increase, all peaking 1-3 hours post injection, except for IL-5 (Figure 8C). IL-5 showed a delayed peak 3-8 hours post injection (Figure 8C). These were the same cytokines that have been indicated to increase in some patients in the clinical studies with BI-1206.

### Premedication

In order to investigate if premedication with corticosteroids could inhibit the IRRs and associated toxicities of AT-130-2, mice were premedicated with 40 mg/kg betamethasone 16-24h and 1h pre injection of AT-130-2. Both the IRRs and the platelet decrease seen with AT-130-2 was completely inhibited with premedication (Figure 9A-B). Also, the increase in liver transaminases and cytokine release was less profound (Figure 9C and data not shown). The same effect was seen when another corticosteroid, dexamethasone was assessed (data not shown).

To assess the importance of the dose of corticosteroid treatment the dose of betamethasone was decreased from 40 mg/kg to 10 mg/kg in the following experiment (Figure 10). At 10 mg/kg both IRRs and a decrease in platelet count were seen in half of the mice indicating that a high dose of corticosteroids is needed to completely block the IRRs and associated toxicities (Figure 10).

Further, the importance of the two doses of corticosteroid treatment was investigated by comparing the protective effect of only early (1 hours pre injection) or only late (24 hours pre injection) premedication with the two doses of corticosteroid treatment. Premedication only 1 hour pre injection could not inhibit the IRRs nor the decrease in platelet count (Figure 11). Premedication 24h pre injection diminished the IRRs and the platelet decrease but could not completely block these symptoms (Figure 11), indicating that two doses of corticosteroids is needed to fully block IRRs.

Finally, the impact of antihistamine, which is a standard premedication in the clinical trials, was evaluated. Premedication with antihistamine alone did not inhibit IRRs or platelet decrease. When combining the two doses of corticosteroid treatment with anti-histamine pretreatment the protective effect was retained (Figure 12). These results were confirmed for three different types of antihistamines (Zyrlex, Zantac and Aeurius).

### Conclusions

Our data demonstrates an in vivo model using intra-venous (i.v.) or intra-perinatal (i.p.) administration of anti-FcyRIIB mlgG2a surrogate (AT-130-2) in wild type mice recapitulates the tolerability profile seen with BI-1206, including IRR's, decreased platelet count, elevated transaminases (i.e. ALT and AST) and transient cytokine release. The IRR's appear 5-20 minutes after AT-130-2 injection with macroscopic symptoms including isolation, decreased activity, impaired balance, piloerection, hunching followed by un-natural body posture and decreased blood pressure. The visual physical reaction is transient, and the animals are fully recovered 1h after the antibody administration. The macroscopic symptoms are accompanied by a decrease in platelet count and elevated transaminases which are normalized within 8 hours. The cytokine release is acute and transient and includes IL-6, IL-5, IL-10, TNFα and KC/GRO (rodent homolog of human IL-8). The cytokine profile and kinetics is equivalent to what is seen after BI-1206 in human subjects. In the mouse model there is an apparent correlation between the IRR's and high and rapid exposure, rather than time of FcyRIIB saturation, where sub-cutaneous (s.c.) administration of AT-130-2 is better tolerated than i.p. and i.v. administration. The timing of onset of symptoms correlates with the serum concentration where receptor saturation is achieved. However, also when administrating an antibody dose that achieves receptor saturation for 6 days or longer the animals recover from all symptoms within 24h. Sustained FcyRIIB blockade per se does not appear to be the causative of IRRs.

In this model premedication with two doses of corticosteroids (dexamethasone or betamethasone) inhibits the macroscopic IRRs as well as platelet decrease and transaminase elevation. The two doses are given s.c. 16-24 hours and i.v. 30-60 minutes prior to antibody administration. The prevention of macroscopic symptoms in the mice by corticosteroids is dose-dependent and importantly the timing of premedication is crucial. The dose 16-24 hours prior to antibody administration is imperative in order to gain the protective effect. If corticosteroids are only given 30-60 minutes prior to antibody administration no protective effect with regard to the macroscopic symptoms is seen, whereas the dose16-24 hours prior to antibody administration alone partially improves tolerability. When both doses are given, inhibition of the macroscopic symptoms, platelet decrease, transaminase elevation, and cytokine release is achieved.

### Example 2 - Other targets

### Materials & Methods

### Test and control substances

The anti-mouse CD32b clone was transiently expressed in HEK293 cells. The specificity of the batch was demonstrated in a luminescence-based enzyme linked immunosorbent assay (ELISA) or in flow cytometry analyses. Endotoxin-levels of antibodies were found to be <0.1 IU/mL as determined by the LAL-Amoebocyte test. The anti-mouse CD40, EGFR and CSFR1 antibodies were purchased from BioXcell or Absolute Antibody (see table below) and the anti-mouse FcγRIII antibody AT154-2 was a gift from University of Southampton. Alternatively, AT154-2 as rat IgG2b isotype may be purchased from, for example, BioRad, Argio Biolaboratories (ARG23942) or LSBio (LS-C745656) which is then converted into IgG2a format using any well-known method.

| **Antibody clone** | **Description** | **Reference** |
|---|---|---|
| AT-130-2 Mouse IgG2a | anti-mouse CD32b | (*See comments above Example 1)* |
| FGK4.5/ FGK45 Rat IgG2a | anti-mouse CD40 | BP0016-2, BioXcell, |
| 7A7 Mouse IgG2a | anti-mouse EGFR | Ab00134-2.0, Absolute Antibody |
| AFS98 Rat IgG2a | anti-mouse CSFR1 | BE0213, BioXcell |
| AT154-2 Mouse IgG2a | anti-mouse FcγRIII | *(See comments above the table)* |

### Mice

Six to eight weeks-old (17-20 g) female C57/BL6 mice were obtained from Taconic. Mice were injected intra-venous (i.v.), with 200 µg/mouse of the different antibodies.

### Premedication

For the corticosteroid treatment, Betapred (betamethasone, VNR: 008938, Alfa-sigma S.P.A.) or Dexamethasone (Cat. No: S1322, Batch No: 02, Selleckchem) was used.

### Animal monitoring

Mice were monitored post injection with regard to changes in behavior and macroscopic symptoms such as isolation, mobility, and fur condition. Macroscopic IRRs scoring system of 0-2 was set up based on the observations:

| **Scoring** | **Macroscopic symptoms** |
|---|---|
| 0 | No visible symptoms |
| 1 | Isolation, decreased activity |
| 2 | Isolation, decreased activity, impaired balance, piloerection, hunching followed by un-natural body posture |

### Blood sampling

Blood samples were collected from vena saphena for instant blood count analysis.

### Platelet count

Platelet counts were analyzed in fresh blood using a Vetscan (Vetscan HM5 Abaxis, Triolab).

### Conclusions

This example shows that the model described herein can distinguish between antibody molecules that induce tolerability issues and those that do not. It further shows that premedication can inhibit IRRs related to different antibodies and targets.

This example also shows that antibodies that induce IRRs also induce thrombocytopenia. Furthermore, it demonstrates that premedication can inhibit thrombocytopenia related to different antibodies and targets.

## Claims

1. A method for predicting if a therapeutic antibody molecule binding specifically to a human target will be associated with a tolerability issue in connection with intravenous administration to a human, comprising the following step:
(i) intravenous or intraperitoneal administration of the therapeutic antibody molecule, if cross-reactive with murine target, or a surrogate antibody, to a mouse and observation of the mouse during a period following immediately after the administration of the therapeutic or surrogate antibody, wherein a display of the macroscopic symptoms isolation and decreased activity during the period followed by restoration of the state of the mouse to the normal state is an indication that the intravenous administration of the therapeutic antibody molecule to a human will be associated with a tolerability issue,
and/or for predicting if a prophylactic or therapeutic treatment, an altered administration route and/or a modification of the therapeutic antibody molecule can prevent or mitigate a tolerability issue associated with intravenous administration to a human of a therapeutic antibody molecule binding specifically to a human target,
comprising the following step(s) in addition to (i) as set out above:
(ii) administration of a prophylactic or therapeutic agent to a mouse in conjunction with intravenous or intraperitoneal administration of the therapeutic or surrogate antibody to a mouse, and observation of the mouse during a period following immediately after the administration of the therapeutic or surrogate antibody, wherein a decreased display of the macroscopic symptoms compared to the macroscopic symptoms displayed by the mouse in (i) or no display of the macroscopic symptoms during the period is an indication that pre-treatment with the prophylactic or therapeutic agent in combination with administration of the therapeutic antibody molecule to a human can prevent or mitigate the tolerability issue that otherwise would be associated with intravenous administration of the therapeutic antibody molecule to a human;
(iii) administration of the therapeutic or surrogate antibody to a mouse by a route of administration other than intravenous or intraperitoneal administration, and observation of the mouse during a period following immediately after the administration of the therapeutic or surrogate antibody, wherein a decreased display of the macroscopic symptoms compared to the macroscopic symptoms displayed by the mouse in (i) or no display of the macroscopic symptoms during the period is an indication that the other route of administration can be used for administration of the therapeutic antibody molecule to a human to prevent or mitigate the tolerability issue that would be associated with intravenous administration of the therapeutic antibody molecule to a human; and/or
(iv) intravenous or intraperitoneal administration of a modified format of the therapeutic or surrogate antibody to a mouse by a route of administration other than intravenous or intraperitoneal administration, and observation of the mouse during a period following immediately after the administration of the modified therapeutic or surrogate antibody, wherein a decreased display of the macroscopic symptoms compared to the macroscopic symptoms displayed by the mouse in (i) or no display of the macroscopic symptoms during the period is an indication that administration of the therapeutic antibody molecule in the modified format to a human can be used to prevent or mitigate the tolerability issue that would be associated with intravenous administration of the therapeutic antibody molecule to a human.

2. A method according to claim 1, wherein a display in (i) of 1-3 additional macroscopic symptoms selected from impaired balance, piloerection, and hunching followed by un-natural body posture during the period in (i) where after the state of the mouse is restored to the normal state further strengthens the indication that the intravenous administration of the therapeutic antibody molecule to the human will be associated with a tolerability issue.

3. A method according to claim 1 or 2, wherein the period during which the macroscopic symptoms are displayed in (i) starts 5-10 minutes after administration of the therapeutic or surrogate antibody and ends 45-90 minutes after administration of the therapeutic or surrogate antibody, and wherein the observation period in (ii), (iii) and/or (iv) is of the same length.

4. A method according to any one of the claims 1-3, wherein at least one of the following additional parameters:
• decreased blood pressure
• decreased platelet count, and or
• increased liver enzymes (AST/ALT).
observed during the period in (i) further strengthens the indication that the intravenous administration of the therapeutic antibody molecule to a human will be associated with a tolerability issue.

5. A method for predicting if a prophylactic or therapeutic treatment can prevent or mitigate a tolerability issue associated with intravenous administration to a human of a therapeutic antibody molecule binding specifically to a human target according to any one of the claims 1-4 comprising at least steps (i) and (ii), wherein pre-treatment is used in (ii) and wherein this pre-treatment is administration of a corticosteroid to the mouse prior to injection of the therapeutic or surrogate antibody.

6. A method according to claim 5, wherein the pre-treatment comprises two administrations of a corticosteroid, wherein one is given 10-48 hours prior to administration of the therapeutic or surrogate antibody and the other is given 5 minutes - 5 hours prior to administration of the therapeutic or surrogate antibody.

7. A method according to claim 6, wherein the corticosteroid is dexamethasone or betamethasone.

8. A method for predicting if an altered administration route can prevent or mitigate a tolerability issue associated with intravenous administration to a human of a therapeutic antibody molecule binding specifically to a human target according to any one of the claims 1-7 comprising at least steps (i) and (iii), wherein the route of administration used in (iii) is subcutaneous administration.

9. A method for predicting if a modification of the therapeutic antibody molecule can prevent or mitigate a tolerability issue associated with intravenous administration to a human of a therapeutic antibody molecule binding specifically to a human target according to any one of the claims 1-8 comprising at least steps (i) and (iv), wherein the modified format of the therapeutic or surrogate antibody used in (iv) is a modification that leads to decreased or abolished engagement of Fc receptors.

10. A method according to any one of the claims 1-9, wherein the human target is selected from the group consisting of FcγRIIB, FcγRIIA and CD40.

11. A method according to claim 10, wherein the therapeutic antibody molecule is a human anti-FcγRIIB antibody capable of binding a human FcγR via its Fc domain and wherein the mouse surrogate antibody is an anti-FcγRIIb antibody capable of binding a mouse FcγR via its Fc domain.

12. A method according to claim 11, wherein the therapeutic antibody molecule is a human anti-FcγRIIB IgG1 antibody and wherein the mouse surrogate antibody is an anti-FcγRIIb mlgG2a.

13. A corticosteroid for use in a dosing regimen to prevent or mitigate a tolerability issue in connection with intravenous administration of a therapeutic antibody molecule to a subject,
wherein the therapeutic antibody molecule has been predicted to be associated with a tolerability issue in connection with intravenous administration to a human using the method of any one of the claims 1-12, and/or wherein pre-treatment with the corticosteroid combination with administration of the therapeutic antibody molecule to a human has been predicted to prevent or mitigate the tolerability issue that otherwise would be associated with intravenous administration of the therapeutic antibody molecule to a human using the method of any one of the claims 5-7, or any one of the claims 10-12 when referring to any one of the claims 5-7,
and wherein the dosing regimen comprises administration of the corticosteroid to the subject in at least two doses prior to intravenous administration of the therapeutic antibody molecule, wherein one dose of the corticosteroid is administered 10-48 hours prior to start of the administration of therapeutic antibody molecule ("the first dose") and one dose of the corticosteroid is administered 5 minutes - 5 hours prior to the start of administration of the therapeutic antibody molecule ("the second dose").

14. A corticosteroid for use according to claim 13, wherein the first dose is given 6-36 hours prior to start of administration of the therapeutic antibody molecule and the second dose is given 15-120 minutes prior to start of administration of the therapeutic antibody molecule.

15. A corticosteroid for use according to claim 13 or 14, wherein the first dose is given 16-24 hours prior to start of administration of the therapeutic antibody molecule.

16. A corticosteroid for use according to any one of the claims 13-15, wherein the second dose is given 30-60 minutes prior to start of administration of the therapeutic antibody molecule.

17. A corticosteroid for use according to any one of the claims 13-16, wherein the dosing regimen comprises administration of the at least two doses of the corticosteroid prior to each infusion of the antibody during the course of antibody therapy.

18. A corticosteroid for use according to claim any one of the claims 13-17, wherein the corticosteroid is dexamethasone or betamethasone or a combination of dexamethasone and betamethasone.

19. A corticosteroid for use according to any one of the claims 13-18, wherein the corticosteroid is dexamethasone and wherein the first dose is 4-20 mg and the second dose is 4-25 mg.

20. A corticosteroid for use according to claim 19, wherein the first dose is 10-12 mg and the second dose is 20 mg.

21. A corticosteroid for use according to any one of the claims 13-18, wherein the corticosteroid is betamethasone and wherein the first dose is 3.2-16 mg and the second dose is 3.2-20 mg.

22. A corticosteroid for use according to claim 21, wherein the first dose is 8-9.6 mg and the second dose is 16 mg.

23. A corticosteroid for use according to claim any one of the claims 13-22, wherein the dosing regimen further comprises administration of an antihistamine 10 minutes - 24 hours prior to start of administration of the therapeutic antibody molecule.

24. A corticosteroid for use according to any one of the claims 13-23, wherein the therapeutic antibody is an Fc receptor binding antibody.

25. A corticosteroid for use according to any one of the claims 13-24, wherein the therapeutic antibody is an anti-FcγRIIB antibody.

26. A corticosteroid for use according to claim 25, wherein the anti-FcγRIIB antibody is the antibody having a light chain with SEQ ID No: 1 and a heavy chain with SEQ ID No: 2.

27. A therapeutic antibody molecule for use in the treatment of cancer, wherein the therapeutic antibody molecule has been predicted to be associated with a tolerability issue in connection with intravenous administration to a human using the method of any one of the claims 1-12 and/or wherein the subcutaneous route of administration of therapeutic antibody molecule to a human has been predicted to prevent or mitigate the tolerability issue that otherwise would be associated with intravenous administration of the therapeutic antibody molecule to a human using the method of claim 8, or any one of the claims 10-12 when referring to claim 8,
and wherein the therapeutic antibody is formulated for subcutaneous administration.

28. A therapeutic antibody molecule for use according to claim 27, wherein the therapeutic antibody is an anti-FcγRIIB antibody.

29. A therapeutic antibody molecule for use according to claim 28, wherein the anti-FcγRIIB antibody is the antibody having a light chain with SEQ ID No: 1 and a heavy chain with SEQ ID No: 2.

30. A modified format of a therapeutic antibody molecule for use in the treatment of cancer, wherein the therapeutic antibody molecule has been predicted to be associated with a tolerability issue in connection with intravenous administration to a human using the method of any one of the claims 1-12 and/or wherein administration of the therapeutic antibody molecule in the modified format to a human has been predicted to prevent or mitigate the tolerability issue that otherwise would be associated with intravenous administration of the therapeutic antibody molecule to a human using the method of claim 9, or any one of the claims 10-12 when referring to claim 9,
and wherein the therapeutic antibody molecule is an Fc receptor binding antibody and the modified format is an antibody having the same Fv variable sequence but having impaired or abrogated FcγR binding compared with the therapeutic antibody molecule.

31. A modified format of a therapeutic antibody molecule for use according to claim 31, wherein the therapeutic antibody is an anti-FcγRIIB antibody.

32. A modified format of a therapeutic antibody molecule for use according to claim 31, wherein the modified format of the anti-FcγRIIB antibody is the antibody having a light chain with SEQ ID No: 1 and a heavy chain with SEQ ID No: 3.
